# EUROPEAN PATENT APPLICATION

(11) **EP 4 790 063 A1**
(43) Date of publication of application: **12.08.2026**
(21) Application number: 24874755.2
(22) Date of filing: 04.10.2024
(51) Int. Cl.: C07K 5/097, A01N 43/36, A01P 7/00, A01P 9/00, C07K 5/078

(54) **PEPTIDE HAVING ACTIVITY FOR INHIBITING ADHESION OF MARINE FOULING**

(30) Priority: 06.10.2023 JP 2023174771
(71) Applicant: National University Corporation Hokkaido University, Hokkaido 060-0808 (JP); Central Research Institute of Electric Power Industry, Chiyoda-ku Tokyo 100-8126 (JP)
(72) Inventor: UMEZAWA, Taiki, Sapporo-shi, Hokkaido 060-0808 (JP); NOGATA, Yasuyuki, Tokyo 100-8126 (JP)
(74) Representative: J A Kemp LLP
(86) International application number: PCT/JP2024/035636
(87) International publication number: WO 2025/075155

(57) **Abstract**

Compounds synthesized using amino acids that are readily available in the market and can be used industrially, which have an antifouling effect against marine fouling organisms and low toxicity are provided. The present invention relates to a composition comprising a dipeptide containing a proline residue, or a tripeptide containing a proline residue and a phenylalanine residue represented by formula (1): 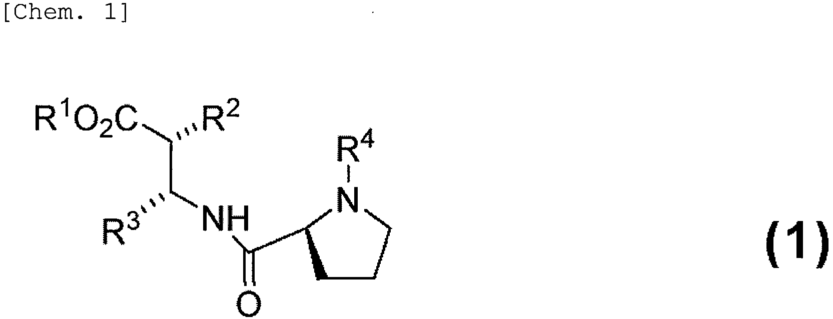 in which
R1 is a substituted or unsubstituted C3 to C10 hydrocarbon group; R2 and R3 are a hydrogen atom or a substituted or unsubstituted C1 to C8 hydrocarbon group; and R4 is a hydrogen atom, a substituted or unsubstituted C1 to C6 hydrocarbon group, and so on.

## Description

### TECHNICAL FIELD

The patent application claims the priority and benefits under the Paris Convention with respect to the Japanese Patent Application No. 2023-174771 filed on October 6, 2023, and the priority under Article 41 of the Japanese Patent Law, and the entirety of the content described in the above application is incorporated herein by reference.

The present invention relates to a compound for preventing the adhesion of marine fouling organisms and to a composition comprising the same. More particularly, the present invention relates to a specific dipeptide having proline as a basic skeleton, or a specific tripeptide having a dipeptide composed of proline and phenylalanine as a basic skeleton, and a composition for preventing the adhesion of marine fouling organisms, which comprises the peptide of the present invention.

### BACKGROUND TECHNOLOGY

Marine fouling organisms that adhere to marine structures and ship hulls exist in the ocean, and they cause a deterioration in the fuel efficiency of ships and blockages in cooling pipes of power plants, thereby negatively impacting human activities. Therefore, various antifouling substances have been developed. As antifouling substances, organic lead compounds were used in the past, and organic tin compounds were also used from around the 1960s, however, these are all harmful substances and have a high environmental impact.

In recent years, the development of compositions for preventing the adhesion of marine fouling organisms with low environmental impact has been advancing in both industry and academia. For example, Patent Documents 1 and 2, as well as Non-Patent Documents 1 and 2, propose isocyanate or isothiocyanate compounds as antifouling substances. However, while such isocyanates and isothiocyanates exhibit excellent antifouling actions, they have the issues that they are prone to hydrolysis and are converted into the toxic formamides.

The inventors of the present application have also developed environmentally friendly antifouling substances, and confirmed that glucosamine derivatives exhibit strong antifouling actions against the cypris larvae of striped barnacle (*Amphibalanus amphitrite, syn. Balanus amphitrite*). However, they do not show any antifouling actions against Mediterranean mussel (*Mytilus galloprovincialis*), also known as blue mussel and there were doubts about their applicability to a wide range of fouling organisms (Non-patent Document 3).

Dolastatin 16 represented by formula (a): 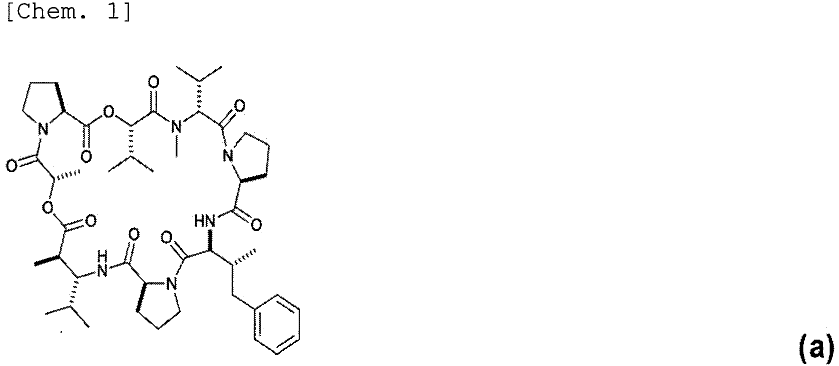 is a natural organic compound isolated from a type of sea hare, Dolabella auricularia, and it is a cyclic depsipeptide containing two unusual amino acids (dolaphenvaline and dolamethylleuine). The isolated Dolastatin 16 has been confirmed to have a growth-inhibiting effect on NCI-H460 (human lung cancer cell line), KM20L2 (human colon cancer cell line), and SF-295 (human glioblastoma cell line) (Non-Patent Document 4). Further, Dolastatin 16 was shown to have antifouling actions against the cypris larvae of the striped barnacle (Non-Patent Document 5).

The present inventors focused on the antifouling action of Dolastatin 16, and carried out the total synthesis of Dolastatin 16, thereby successfully synthesizing the two unusual amino acids contained in Dolastatin 16: dolaphenvaline and dolamethylleuine (Non-Patent Document 6).

Further, the present inventors successfully achieved the total synthesis of Dolastatin 16, and also confirmed that the synthetic Dolastatin 16 exhibits an antifouling effect equivalent to that of the natural Dolastatin 16 (Non-Patent Document 7). Additionally, the present inventors found that the two peptides, represented by formulas (b) and (c), which serve as precursors for the total synthesis of Dolastatin 16, also exhibit excellent antifouling effects.

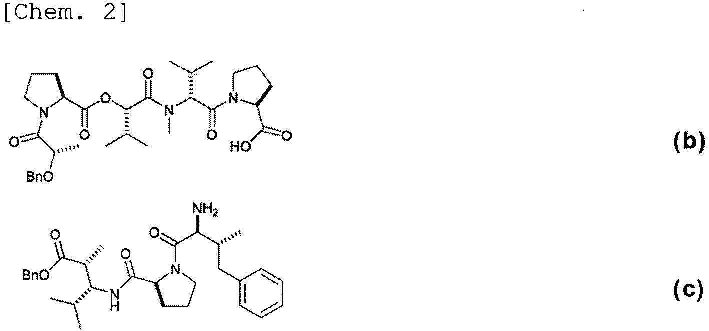

Although the synthetic Dolastatin 16 is a promising antifouling substance, its preparation requires a complex synthetic route. Therefore, recognizing the promising potential of the two peptides mentioned above as antifouling substances, the present inventors synthesized a tripeptide containing the two unusual amino acids, dolaphenvaline and dolamethylleuine, contained in Dolastatin 16, and investigated its antifouling effects (Non-Patent Document 8). The antifouling effect of tripeptide 3 as represented by the following formula (EC₅₀ = 1.17 µg/mL), improved to EC₅₀ = 0.79 µg/mL when the amino group was modified with tert-butoxy carbonyl group (Boc group) to form tripeptide Boc-3.

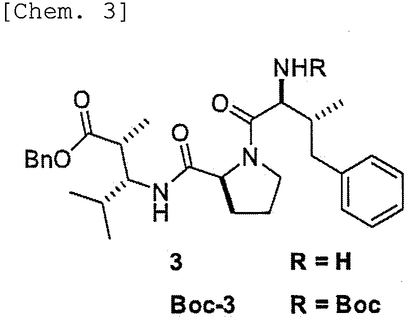

### CITATION LIST

### PATENT DOCUMENTS

Patent Document 1: Japanese Publication No. 2015-042622
Patent Document 2: Japanese Publication No. 2019-167335

### NON-PATENT DOCUMENTS

Non-Patent Document 1: Fukuda, T. et al., Chem. Biodivers., 2016, 13, 1502
Non-Patent Document 2: Tanikawa, A. et al., Chem. Biodivers., 2023, 20, e202200953
Non-Patent Document 3: Umezawa, T. et al., Mar. Drugs, 2017, 15, 203; doi:10.3390/md15070203
Non-Patent Document 4: Pettit, G.R. et al., J. Nat. Prod. 1997, 60, 752
Non-Patent Document 5: Tan, L.K. et al., Biofouling 2010, 26, 685
Non-Patent Document 6: Umezawa, T. et al., Tetrahedron Letters, 2015, 56, 168
Non-Patent Document 7: Casalme, L.O. et al., Org. Biomol. Chem., 2017, 15, 1140
Non-Patent Document 8: Casalme, L.O. et al., Mar. Drugs 2022, 20, 124

### SUMMARY OF THE INVENTION

### PROBLEMS TO BE SOLVED BY THE INVENTION

The present inventors have discovered that a tripeptide containing the two unusual amino acids, dolaphenvaline and dolamethylleuine, which are contained in Dolastatin 16, exhibits an antifouling effect against fouling organisms present in the ocean. However, the synthesis of such tripeptides is extremely costly, and involves issues with large-scale supply, due to use of unusual amino acids, which are not commercially available.

Under the circumstances, the present invention aims to synthesize a tripeptide that exhibits an antifouling effect and low toxicity, using inexpensive amino acids that are readily available in the market and easy to use industrially.

### MEANS TO SOLVE PROBLEMS

The inventors of the present application diligently conducted research and found that tripeptides composed solely of α-amino acids did not exhibit any antifouling effects, whereas dipeptides and tripeptides containing β-amino acids exhibited antifouling effects against the cypris larvae of Striped Barnacle. The inventors also found that some tripeptides showed anti-fouling activity against Mediterranean mussel.

Moreover, the peptides of the present invention should be stable against hydrolysis in seawater, and can be expected to have a moderate biodegradability through enzymes and the like, since they are biomolecules. In other words, the degradation products derived from the compounds of the present invention after they have demonstrated the antifouling actions for appropriate periods, and then undergone biodegradation and flowed into the ocean, can be also expected to exhibit extremely minimal adverse effects on the ecosystem.

Specifically, the present invention encompasses the aspects as follows.

### <Dipeptides and tripeptides>

[1] A dipeptide containing a proline residue, or a tripeptide containing a proline residue and a phenylalanine residue represented by formula (1): 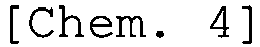 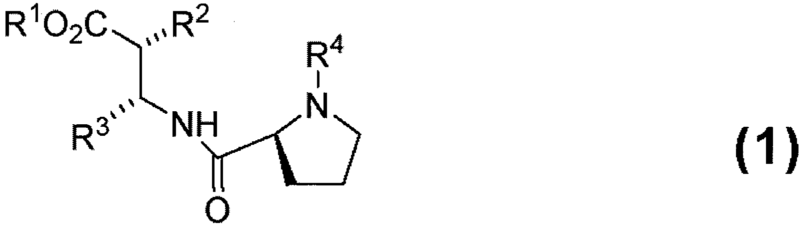 in which
   R1 is a substituted or unsubstituted C3 to C10 hydrocarbon group;
   R2 and R3 are a hydrogen atom or a substituted or unsubstituted C1 to C8 hydrocarbon group, or R2 and R3 together with the adjacent carbon atoms form a substituted or unsubstituted cyclic C1 to 10 hydrocarbon group;
   R4 is a hydrogen atom, a substituted or unsubstituted C1 to C6 hydrocarbon group, a carboxyl group or alkoxy acyl group represented by formula (2): 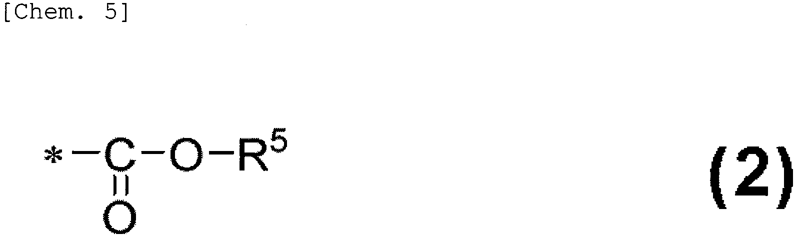 in which
   R5 is a hydrogen atom or a C1 to C6 hydrocarbon group, and the symbol * indicates the bonding part with the pyrrolidine group, or a phenylalanine residue represented by formula (3) or (3'); 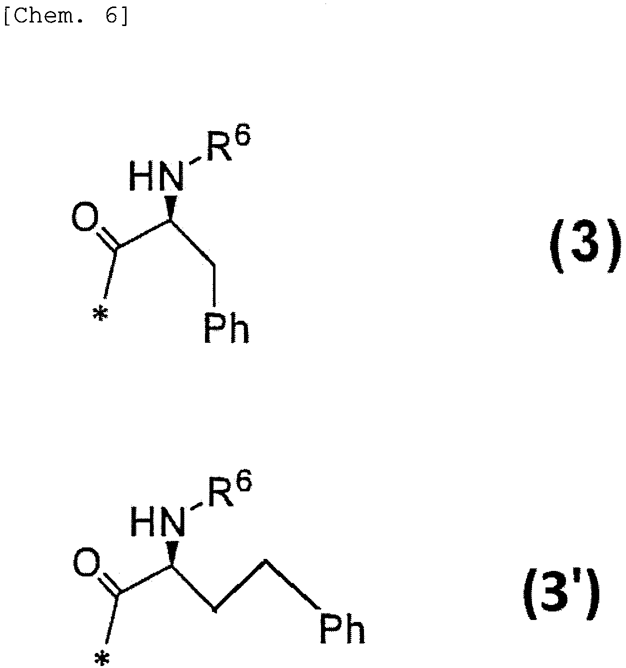 in which
   R6 is a hydrogen atom, a C1 to C6 hydrocarbon group, or a carboxyl group or alkoxy acyl group represented by formula (2), Ph is a phenyl group, and the symbol * indicates the bonding part with the pyrrolidine group;
   provided that compounds in which R1 is a benzyl group, both R2 and R3 are phenyl groups, and R4 is a tert-butoxycarbonyl group are excluded.
[2] The dipeptide containing the proline residue, or the tripeptide containing the proline residue and the phenylalanine residue according to [1], wherein the cumulative carbon number in substituents: R1, R2, and R3 is 7 or more, preferably 9 or more, and more preferably 11 or more.
[3] The dipeptide containing the proline residue, or the tripeptide containing the proline residue and the phenylalanine residue according to [1] or [2],
   in which
   the substituted or unsubstituted C3 to C10 hydrocarbon group in R1 is selected from the group consisting of a substituted or unsubstituted, linear or branched C3 to C10 alkyl group; a substituted or unsubstituted, linear or branched C3 to C10 alkenyl group; a substituted or unsubstituted cyclic C3 to C10 alkyl group; a substituted or unsubstituted cyclic C3 to C10 alkenyl group; and a substituted or unsubstituted C3 to C10 alkyl-substituted aryl group;
   the substituted or unsubstituted C1 to C8 hydrocarbon groups in R2 and R3 are independently selected from the group consisting of a substituted or unsubstituted, linear or branched C1 to C8 alkyl group; a substituted or unsubstituted, linear or branched C1 to C8 alkenyl group; a substituted or unsubstituted cyclic C1 to C8 alkyl group; a substituted or unsubstituted cyclic C1 to C8 alkenyl group; and a substituted or unsubstituted C1 to C8 alkyl-substituted aryl group, and, when R2 and R3 together with the adjacent carbon atoms form a substituted or unsubstituted cyclic C1 to C10 hydrocarbon group, the cyclic C1 to C10 hydrocarbon group is selected from the group consisting of a substituted or unsubstituted cyclic C1 to C10 alkyl group; a substituted or unsubstituted cyclic C1 to C10 alkenyl group; and a substituted or unsubstituted C1 to C10 alkyl-substituted aryl group;
   the substituted or unsubstituted C1 to C6 hydrocarbon group in R4 is selected from the group consisting of a substituted or unsubstituted, linear or branched C1 to C6 alkyl group; a substituted or unsubstituted, linear or branched C1 to C6 alkenyl group; a substituted or unsubstituted cyclic C1 to C6 alkyl group; a substituted or unsubstituted cyclic C1 to C6 alkenyl group; and a substituted or unsubstituted C1 to C6 alkyl-substituted aryl group; and
   the substituted or unsubstituted C1 to C6 hydrocarbon groups at R5 and R6 are independently selected from the group consisting of a substituted or unsubstituted, linear or branched C1 to C6 alkyl group; a substituted or unsubstituted, linear or branched C1 to C6 alkenyl group; a substituted or unsubstituted cyclic C1 to C6 alkyl group; a substituted or unsubstituted cyclic C1 to C6 alkenyl group; and a substituted or unsubstituted phenyl group.
[4] The dipeptide containing the proline residue, or the tripeptide containing the proline residue and the phenylalanine residue according to any one of [1] to [3], in which
   the substituted or unsubstituted C3 to C10 hydrocarbon group in R1 is selected from the group consisting of a linear or branched alkyl group selected from the group consisting of propyl group, butyl group, pentyl group, hexyl group, heptyl group, octyl group, nonyl group, and decyl group; a linear or branched alkenyl group selected form the group consisting of propenyl group, butenyl group, pentenyl group, hexenyl group, heptenyl group, octenyl group, nonenyl group, and decenyl group; a cyclic alkyl group selected from the group consisting of cyclopropyl group, cyclobutyl group, cyclopentyl group, cyclohexyl group, cycloheptyl group, cyclooctyl group, cyclononyl group, and cyclodecyl group; a cyclic alkenyl group selected from the group consisting of cyclopropenyl group, cyclobutenyl group, cyclopentenyl group, cyclohexenyl group, cycloheptenyl group, cyclooctenyl group, cyclononenyl group, and cyclodecenyl group; an aryl group selected from the group consisting of phenyl group and naphthyl group, an alkyl-substituted phenyl group selected from tolyl group, ethylphenyl group, xylyl group, and benzyl group;
   the C1 to C8 hydrocarbon group of the substituted or unsubstituted C1 to C8 hydrocarbon group in R2 and R3 is each independently selected from the group consisting of a linear or branched alkyl group selected from the group consisting of methyl group, ethyl group, propyl group, butyl group, pentyl group, hexyl group, heptyl group, and octyl group; a linear or branched alkenyl group selected from the group consisting of ethenyl group, propenyl group, butenyl group, pentenyl group, hexenyl group, heptenyl group, and octenyl group; a cyclic alkyl group selected from the group consisting of cyclopropyl group, cyclobutyl group, cyclopentyl group, cyclohexyl group, cycloheptyl group, and cyclooctyl group; a cyclic alkenyl group selected from the group consisting of cyclopropenyl group, cyclobutenyl group, cyclopentenyl group, cyclohexenyl group, cycloheptenyl group, and cyclooctenyl group; and an aryl group selected from the group consisting of phenyl group, an alkyl-substituted phenyl groups elected from tolyl group, ethylphenyl group, xylyl group, and benzyl group; and
   the C1 to C6 hydrocarbon group of the substituted or unsubstituted C1 to C6 hydrocarbon group in R5 and R6 is each independently selected from the group consisting of a linear or branched alkyl group selected from the group consisting of methyl group, ethyl group, propyl group, butyl group, pentyl group, and hexyl group; a linear or branched alkenyl group selected from the group consisting of ethenyl group, propenyl group, butenyl group, pentenyl group, and hexenyl group; a cyclic alkyl group selected from the group consisting of cyclopropyl group, cyclobutyl group, cyclopentyl group, and cyclohexyl group; a cyclic alkenyl group selected from the group consisting of cyclopropenyl group, cyclobutenyl group, cyclopentenyl group, and cyclohexenyl group; and phenyl group.

### <Compositions for preventing the adhesion of marine fouling organisms>

[5] A composition for preventing the adhesion of marine fouling organisms, which comprises a dipeptide containing a proline residue, or a tripeptide containing a proline residue and a phenylalanine residue represented by formula (1): 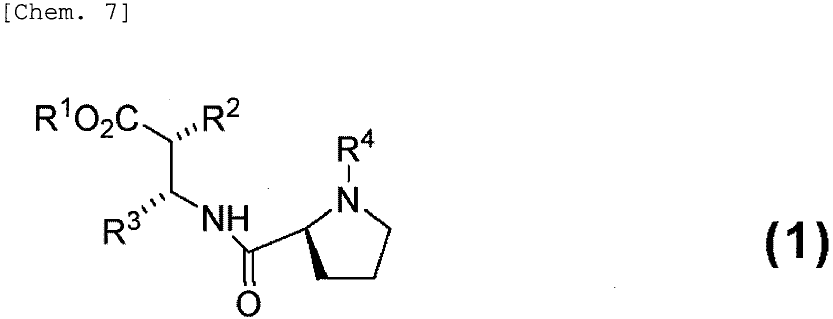 in which
   R1 is a substituted or unsubstituted C3 to C10 hydrocarbon group;
   R2 and R3 are a hydrogen atom or a substituted or unsubstituted C1 to C8 hydrocarbon group, or R2 and R3 together with the adjacent carbon atoms form a substituted or unsubstituted cyclic C1 to 10 hydrocarbon group;
   R4 is a hydrogen atom, a substituted or unsubstituted C1 to C6 hydrocarbon group, a carboxyl group or alkoxy acyl group represented by formula (2): 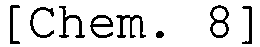 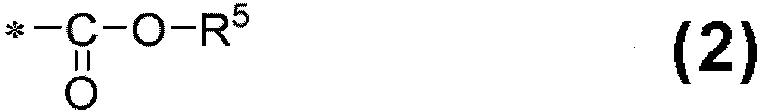 in which
   R5 is a hydrogen atom or a C1 to C6 hydrocarbon group, and the symbol * indicates the bonding part with the pyrrolidine group, or a phenylalanine residue represented by formula (3) or (3'); 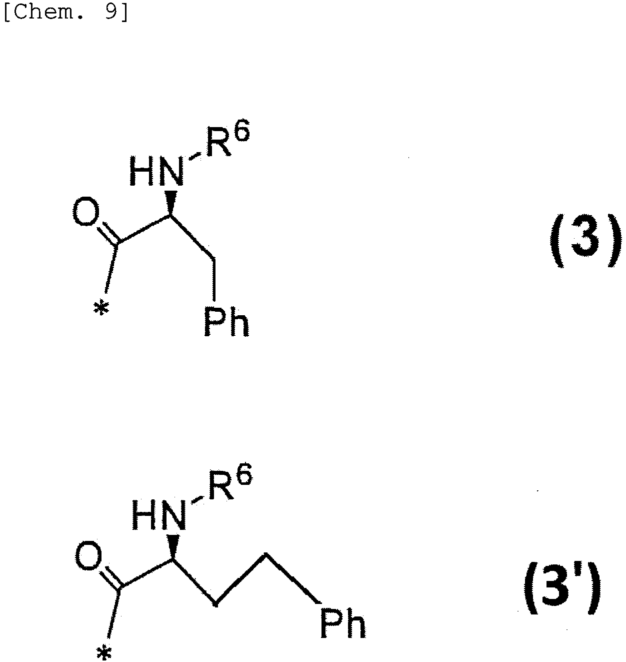 in which
   R6 is a hydrogen atom, a C1 to C6 hydrocarbon group, or a carboxyl group or alkoxy acyl group represented by formula (2), Ph is a phenyl group, and the symbol * indicates the bonding part with the pyrrolidine group;
   provided that the cumulative carbon number in substituents: R1, R2, and R3 is 7 or more, preferably 9 or more, and more preferably 11 or more.
[6] A composition for preventing the adhesion of marine fouling organisms, which comprises a dipeptide containing a proline residue, or a tripeptide containing a proline residue and a phenylalanine residue according to any one of [1] to [4].

### EFFECT OF INVENTION

The present invention has made it possible to easily synthesize antifouling substances possessing antifouling actions against marine fouling organisms and having a minimal adverse impact on the ecosystem from a low-cost amino acid that can be readily obtained in the market and used industrially.

### BRIEF DESCRIPTION OF DRAWINGS

[FIG. 1] The upper part of Figure 1 shows the ¹H NMR spectrum (500 MHz, CDCl₃) of compound 14, while the lower part shows the ¹³C NMR spectrum (126 MHz, CDCl₃) of compound 14.
[FIG. 2] Figure 2 illustrates the data and graphs showing the antifouling activity and toxicity against the cypris larvae of the striped barnacle (results of tripeptides 2 and 3).
[FIG. 3] Figure 3 illustrates the data and graphs showing the antifouling activity and toxicity against the cypris larvae of the striped barnacle (results of tripeptides 4 and 5, as well as copper sulfate).
[FIG. 4] Figure 4 illustrates the data and graphs showing the antifouling activity and toxicity against the cypris larvae of the striped barnacle (results of tripeptides 6 to 8).
[FIG. 5] Figure 5 illustrates the data and graphs showing the antifouling activity and toxicity against the cypris larvae of the striped barnacle (results of tripeptides 9 and 10, as well as dipeptide 11).
[FIG. 6] Figure 6 illustrates the data and graphs showing the antifouling activity and toxicity against the cypris larvae of the striped barnacle (results of tripeptide 12).
[FIG. 7] Figure 7 illustrates the data and graphs showing the antifouling activity and toxicity against the cypris larvae of the striped barnacle (results of dipeptide 13).
[FIG. 8] Figure 8 illustrates the data and graphs showing the antifouling activity and toxicity against the cypris larvae of the striped barnacle (results of dipeptide 14).
[FIG. 9] Figure 9 illustrates the data and graphs showing the antifouling activity and toxicity against the cypris larvae of the striped barnacle (results of copper sulfate).

### ASPECTS FOR CARRYING OUT INVENTION

### <Dipeptides and Tripeptides>

In one aspect, the present invention relates to a dipeptide containing a proline residue, or a tripeptide containing a proline residue and a phenylalanine residue represented by formula (1): 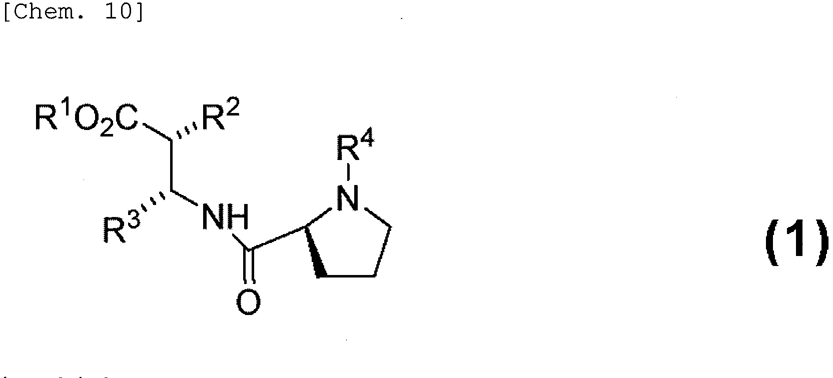 in which
R1 is a substituted or unsubstituted C3 to C10 hydrocarbon group;
R2 and R3 are a hydrogen atom or a substituted or unsubstituted C1 to C8 hydrocarbon group, or R2 and R3 together with the adjacent carbon atoms form a substituted or unsubstituted cyclic C1 to 10 hydrocarbon group;
R4 is a hydrogen atom, a substituted or unsubstituted C1 to C6 hydrocarbon group, a carboxyl group or alkoxy acyl group represented by formula (2): 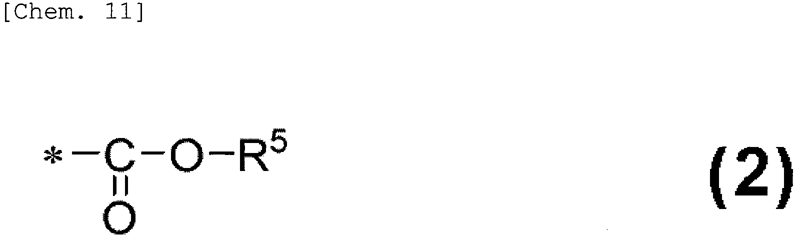 in which
R5 is a hydrogen atom or a C1 to C6 hydrocarbon group, and the symbol * indicates the bonding part with the pyrrolidine group, or a phenylalanine residue represented by formula (3) or (3'); 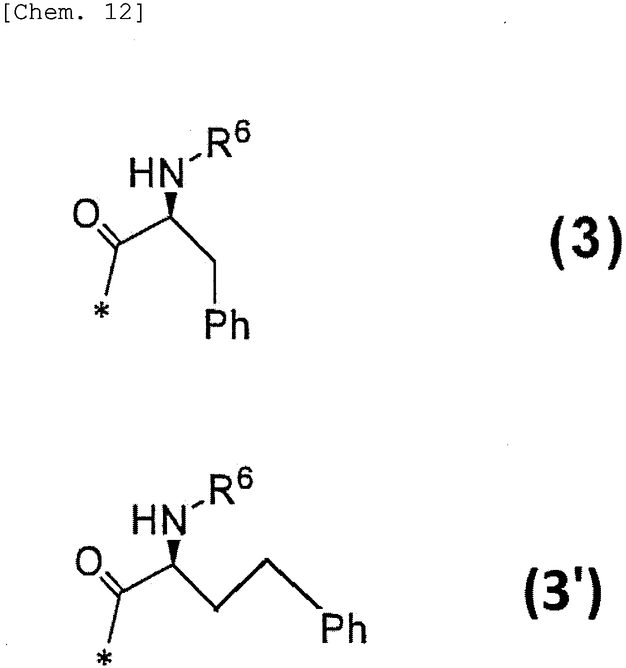 in which
R6 is a hydrogen atom, a C1 to C6 hydrocarbon group, or a carboxyl group or alkoxy acyl group represented by formula (2), Ph is a phenyl group, and the symbol * indicates the bonding part with the pyrrolidine group;
provided that compounds in which R1 is a benzyl group, both R2 and R3 are phenyl groups, and R4 is a tert-butoxycarbonyl group are excluded.

In this regard, the compound being excluded in which R1 is a benzyl group, both R2 and R3 are phenyl groups, and R4 is a tert-butoxycarbonyl group is dipeptide 13 represented by the formula: 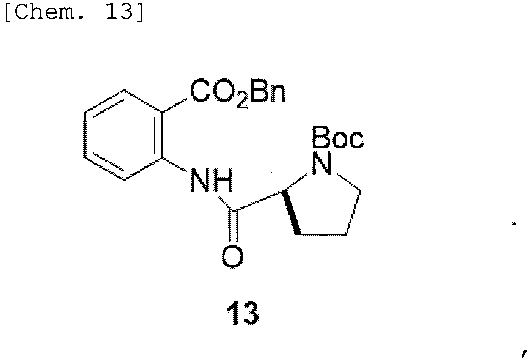 and is described in Reference Example 1. Dipeptide 13 is described in Tetrahedron, 2011, 67, 3969, and it is only mentioned as a model substance for asymmetric reactions, meaning that there is no mention of it as an antifouling substance.

In one embodiment, the present invention relates to the dipeptide containing the proline residue, or the tripeptide containing the proline residue and the phenylalanine residue wherein the definitions of each substituent are the same as above, and wherein the cumulative carbon number in substituents: R1, R2, and R3 is 7 or more, preferably 9 or more, and more preferably 11 or more.

In this form, one embodiment of the present invention relates to the dipeptide containing the proline residue, or the tripeptide containing the proline residue and the phenylalanine residue represented by formula (1),
in which
the substituted or unsubstituted C3 to C10 hydrocarbon group in R1 is selected from the group consisting of a substituted or unsubstituted, linear or branched C3 to C10 alkyl group; a substituted or unsubstituted, linear or branched C3 to C10 alkenyl group; a substituted or unsubstituted cyclic C3 to C10 alkyl group; a substituted or unsubstituted cyclic C3 to C10 alkenyl group; and a substituted or unsubstituted C3 to C10 alkyl-substituted aryl group;
the substituted or unsubstituted C1 to C8 hydrocarbon groups in R2 and R3 are independently selected from the group consisting of a substituted or unsubstituted, linear or branched C1 to C8 alkyl group; a substituted or unsubstituted, linear or branched C1 to C8 alkenyl group; a substituted or unsubstituted cyclic C1 to C8 alkyl group; a substituted or unsubstituted cyclic C1 to C8 alkenyl group; and a substituted or unsubstituted C1 to C8 alkyl-substituted aryl group, and, when R2 and R3 together with the adjacent carbon atoms form a substituted or unsubstituted cyclic C1 to C10 hydrocarbon group, the cyclic C1 to C10 hydrocarbon group is independently selected from the group consisting of a substituted or unsubstituted cyclic C1 to C10 alkyl group; a substituted or unsubstituted cyclic C1 to C10 alkenyl group; and a substituted or unsubstituted C1 to C10 alkyl-substituted aryl group;
the substituted or unsubstituted C1 to C6 hydrocarbon group in R4 is selected from the group consisting of a substituted or unsubstituted, linear or branched C1 to C6 alkyl group; a substituted or unsubstituted, linear or branched C1 to C6 alkenyl group; a substituted or unsubstituted cyclic C1 to C6 alkyl group; a substituted or unsubstituted cyclic C1 to C6 alkenyl group; and a substituted or unsubstituted C1 to C6 alkyl-substituted aryl group; and
the substituted or unsubstituted C1 to C6 hydrocarbon groups at R5 and R6 are independently selected from the group consisting of a substituted or unsubstituted, linear or branched C1 to C6 alkyl group; a substituted or unsubstituted, linear or branched C1 to C6 alkenyl group; a substituted or unsubstituted cyclic C1 to C6 alkyl group; a substituted or unsubstituted cyclic C1 to C6 alkenyl group; and a substituted or unsubstituted phenyl group.

In the present invention, the term "alkyl" refers to a linear or branched hydrocarbon chain consisting of carbon atoms and hydrogen atoms, which does not contain any unsaturation, which contains carbon atoms specified as "C1 to C6" or "C3 to C10", and of which remainder part of the molecule is connected by a single bond. Specifically, it is selected from the group consisting of methyl group, ethyl group, propyl group, butyl group, pentyl group, hexyl group, heptyl group, octyl group, nonyl group, and decyl group.

In the present invention, the term "alkenyl" has the same definition as that of the term "alkyl", except that it contains at least one double bond. Specifically, it is selected from the group consisting of ethenyl group, propenyl group, butenyl group, pentenyl group, hexenyl group, heptenyl group, octenyl group, nonenyl group, and decenyl group.

In the present invention, the term "cyclic alkyl group" has the same definition as that of the term "alkyl," except for it is cyclic. Specifically, it is selected from the group consisting of cyclopropyl group, cyclobutyl group, cyclopentyl group, cyclohexyl group, cycloheptyl group, cyclooctyl group, cyclononyl group, and cyclodecyl group.

In the present invention, the term "cyclic alkenyl group" has the same definition as that of the term "alkenyl", except for it is cyclic, and is specifically selected from the group consisting of cyclopropenyl group, cyclobutenyl group, cyclopentenyl group, cyclohexenyl group, cycloheptenyl group, cyclooctenyl group, cyclononenyl group, and cyclodecenyl group.

In the present invention, the term "aryl group" refers to a cyclic structure comprising at least one aromatic ring, which contains carbon atoms specified as "C1 to C6" or "C3 to C10", and is specifically selected from the group consisting of phenyl group and naphthyl group, an alkyl-substituted phenyl group selected from tolyl group, ethylphenyl group, xylyl group, and benzyl group.

In the present invention, the substituent group involved in "substitution" of "substituted or unsubstituted" is selected from the group consisting of a hydroxyl, a halogen (for example, fluorine, chlorine, bromine), a C1 to C4 alkyl (for example, methyl, ethyl), a C1 to C4 fluoroalkyl (for example, trifluoromethyl), a C1 to C4 alkoxy (for example, methoxy, ethoxy, n-propoxy), a nitro group, an amino group, a N-alkylamino group, a N,N-dialkylamino group, a carboxyl group, an aldehyde group, an acetal group, an alkyl ester group, an alkyl ketone group, a phenyl, and a benzyl.

In one embodiment, the present invention relates to the dipeptide containing the proline residue, or the tripeptide containing the proline residue and the phenylalanine residue represented by formula (1),
in which
the substituted or unsubstituted C3 to C10 hydrocarbon group in R1 is selected from the group consisting of a linear or branched alkyl group selected from the group consisting of propyl group, butyl group, pentyl group, hexyl group, heptyl group, octyl group, nonyl group, and decyl group; a linear or branched alkenyl group selected from the group consisting of propenyl group, butenyl group, pentenyl group, hexenyl group, heptenyl group, octenyl group, nonenyl group, and decenyl group; a cyclic alkyl group selected from the group consisting of cyclopropyl group, cyclobutyl group, cyclopentyl group, cyclohexyl group, cycloheptyl group, cyclooctyl group, cyclononyl group, and cyclodecyl group; a cyclic alkenyl group selected from the group consisting of cyclopropenyl group, cyclobutenyl group, cyclopentenyl group, cyclohexenyl group, cycloheptenyl group, cyclooctenyl group, cyclononenyl group, and cyclodecenyl group; an aryl group selected from the group consisting of phenyl group and naphthyl group, an alkyl-substituted phenyl group selected from tolyl group, ethylphenyl group, xylyl group, and benzyl group;
the C1 to C8 hydrocarbon group of the substituted or unsubstituted C1 to C8 hydrocarbon group in R2 and R3 is each independently selected from the group consisting of a linear or branched alkyl group selected from the group consisting of methyl group, ethyl group, propyl group, butyl group, pentyl group, hexyl group, heptyl group, and octyl group; a linear or branched alkenyl group selected from the group consisting of ethenyl group, propenyl group, butenyl group, pentenyl group, hexenyl group, heptenyl group, and octenyl group; a cyclic alkyl group selected from the group consisting of cyclopropyl group, cyclobutyl group, cyclopentyl group, cyclohexyl group, cycloheptyl group, and cyclooctyl group; a cyclic alkenyl group selected from the group consisting of cyclopropenyl group, cyclobutenyl group, cyclopentenyl group, cyclohexenyl group, cycloheptenyl group, and cyclooctenyl group; and an aryl group selected from the group consisting of phenyl group, an alkyl-substituted phenyl group selected from tolyl group, ethylphenyl group, xylyl group, and benzyl group; and
the C1 to C6 hydrocarbon group of the substituted or unsubstituted C1 to C6 hydrocarbon group in R5 and R6 is each independently selected from the group consisting of a linear or branched alkyl group selected from the group consisting of methyl group, ethyl group, propyl group, butyl group, pentyl group, and hexyl group; a linear or branched alkenyl group selected from the group consisting of ethenyl group, propenyl group, butenyl group, pentenyl group, and hexenyl group; a cyclic alkyl group selected from the group consisting of cyclopropyl group, cyclobutyl group, cyclopentyl group, and cyclohexyl group; a cyclic alkenyl group selected from the group consisting of cyclopropenyl group, cyclobutenyl group, cyclopentenyl group, and cyclohexenyl group; and phenyl group.

Hereinafter, the synthesis method of the compounds of the present invention is generally described.

The dipeptides or the tripeptides represented by formula (1) according to the present invention can be synthesized, for example, through the following steps.

The peptides according to the present invention can be synthesized by reacting a β-amino acid ester represented by formula (4): 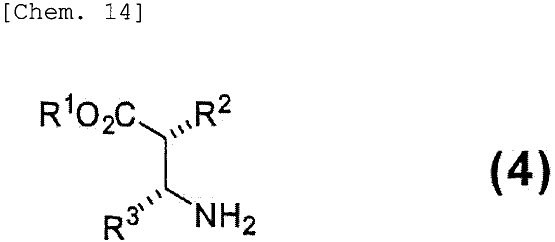 in which
R1 is a substituted or unsubstituted C3 to C10 hydrocarbon group;
R2 and R3 are a hydrogen atom or a C1 to C8 hydrocarbon group, or R2 and R3 together with the adjacent carbon atoms form a substituted or unsubstituted cyclic C1 to 10 hydrocarbon group; and
a monopeptide containing a proline residue or a dipeptide containing a proline residue and a phenylalanine residue represented by formula (5): 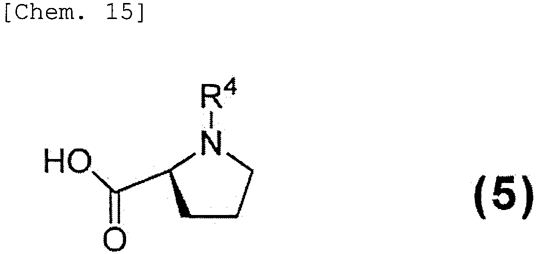 in which
R4 is a hydrogen atom, a C1 to C6 hydrocarbon group, a carboxyl group or an alkoxy acyl group represented by formula (2) : 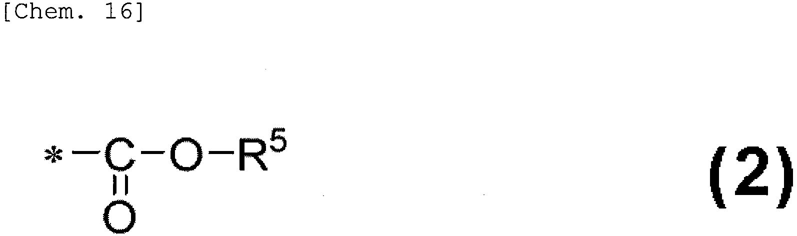 in which
R5 is a hydrogen atom or a C1 to C6 hydrocarbon group, and the symbol * indicates the bonding part with the pyrrolidine group, or a phenylalanine residue represented by formulas (3) or (3'): 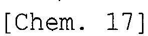 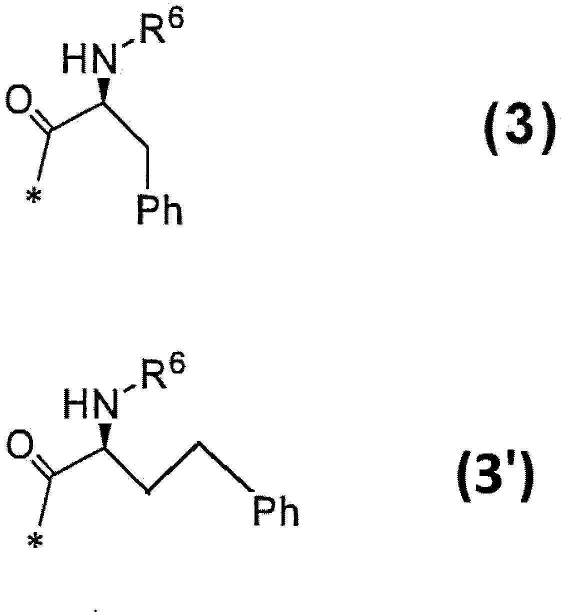 in which
R6 is a hydrogen atom, a C1 to C6 hydrocarbon group, or a carboxyl group or an alkoxy acyl group represented by formula (2), Ph is a phenyl group, and the symbol * indicates the bonding part with the pyrrolidine group;
in an organic solvent.

The organic solvent as used for the synthesis of the oligopeptides of the present invention is preferably one in which the amino acids or peptides that serve as the raw materials and products may be dissolved or dispersed. Solvents or dispersing media that may be used suitably include, but are not limited to, ones that are substantially inert to these reactions and form a liquid or fluid at the reaction temperature. Among these, one in which the amino acids or peptides that serve as the raw materials and products may be dissolved, are particularly preferred.

Specific examples of the organic solvents as used for the synthesis include aliphatic hydrocarbons such as hexane, heptane, octane, cyclohexane, and cyclohexene; aromatic hydrocarbons such as toluene, xylene, ethylbenzene, and naphthalene; linear ethers such as diethyl ether, methyl butyl ether, ethylene glycol dimethyl ether, ethylene glycol diethyl ether, propylene glycol dimethyl ether, and diethylene glycol dimethyl ether; cyclic ethers such as dioxane, tetrahydrofuran, methyl tetrahydrofuran, and dimethylfuran; linear ketones such as acetone, methyl ethyl ketone, and methyl butyl ketone; cyclic ketones such as cyclohexanone; nitriles such as acetonitrile, propionitrile, and benzonitrile; esters such as ethyl acetate, butyl acetate, propylene glycol monomethyl ether acetate, and diethylene glycol monomethyl ether acetate; halogenated hydrocarbons such as methylene chloride, chloroform, carbon tetrachloride, dichloroethane, trichloroethylene, dichloropropane, bromoform, chlorobenzene, and dichlorobenzene; alkyl sulfoxides such as dimethyl sulfoxide; alkyl amides such as dimethylacetamide; supercritical gases such as supercritical carbon dioxide; and others.

### <Compositions for preventing the adhesion of marine fouling organisms>

In another aspect, the present invention relates to a composition for preventing the adhesion of marine fouling organisms, which comprises a dipeptide containing a proline residue, or a tripeptide containing a proline residue and a phenylalanine residue represented by formula (1): 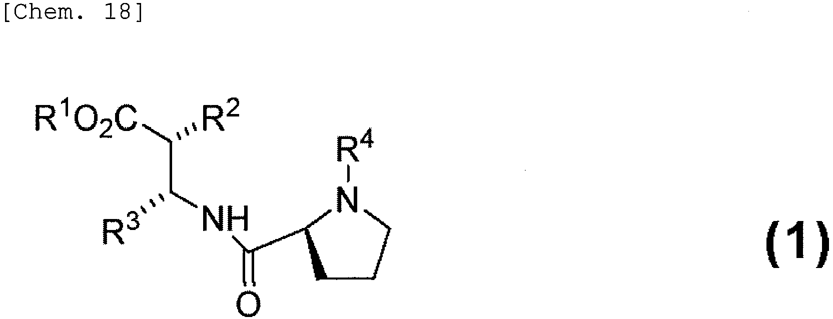 in which
R1 is a substituted or unsubstituted C3 to C10 hydrocarbon group;
R2 and R3 are a hydrogen atom or a substituted or unsubstituted C1 to C8 hydrocarbon group, or R2 and R3 together with the adjacent carbon atoms form a substituted or unsubstituted cyclic C1 to 10 hydrocarbon group;
R4 is a hydrogen atom, a substituted or unsubstituted C1 to C6 hydrocarbon group, a carboxyl group or alkoxy acyl group represented by formula (2): 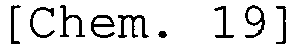 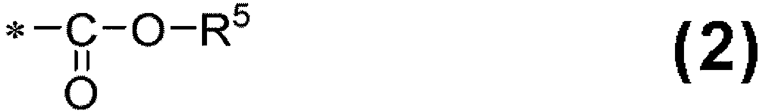 in which
R5 is a hydrogen atom or a C1 to C6 hydrocarbon group, and the symbol * indicates the bonding part with the pyrrolidine group, or a phenylalanine residue represented by formula (3) or (3'); 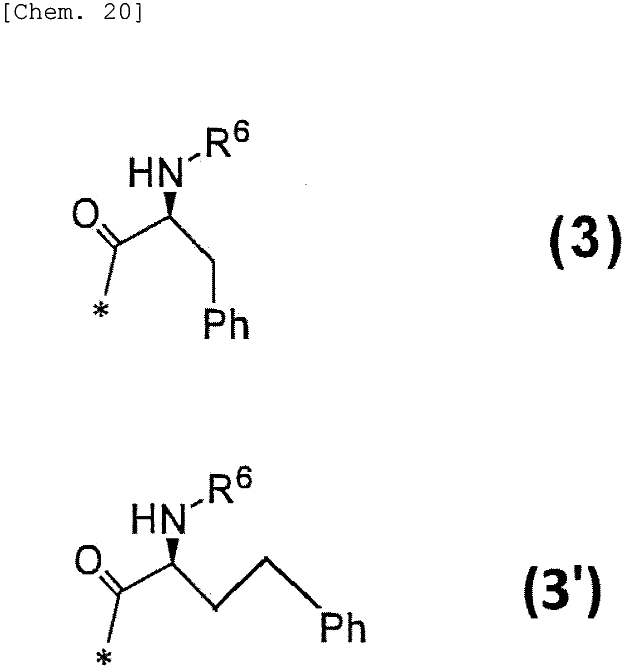 in which
R6 is a hydrogen atom, a C1 to C6 hydrocarbon group, or a carboxyl group or alkoxy acyl group represented by formula (2), Ph is a phenyl group, and the symbol * indicates the bonding part with the pyrrolidine group;
provided that the cumulative carbon number in substituents: R1, R2, and R3 is 7 or more, preferably 9 or more, and more preferably 11 or more.

In this embodiment, from the perspective of the antifouling activity against marine fouling organisms, there may be preferred values for the total number of carbon atoms in the substituents of formula (1): R1, R2, and R3, that is, "cumulative carbon number". The cumulative carbon number of substituents R1, R2, and R3 may be 7 or more and 26 or less, 10 or more and 20 or less, or 11 or more and 16 or less.

In this aspect, one embodiment of the present invention relates to a composition for preventing the adhesion of marine fouling organisms, which comprises a compound of each embodiment of the present invention.

In the present invention, the marine fouling organisms present in the ocean include barnacles, mussels, hydroids, bryozoans, and the like.

The barnacle, after the nauplius larva hatched from the egg grows into the cypris larvae, ends its planktonic life and secretes cement (larval cement) from the tip of the adhesion plate on the first antenna, thereby becoming a juvenile and entering a sessile life. During the growth from the juvenile stage to the adult stage, the organism secretes a cementing substance (adult cement) from the base plate to maintain the adhesion.

On the other hand, the mussels adhere to rocks and ship hulls in the sea using threads of multiple adhesive proteins (byssal threads).

The composition comprising the dipeptide or the tripeptide of the present invention for preventing the adhesion of marine fouling organisms enables to prevent these marine fouling organisms from firmly adhering to the hull.

The composition for preventing the adhesion of marine fouling organisms of the present invention may comprise, in addition to the dipeptide or the tripeptide of the present invention, binders or resins aimed at imparting formability, that is, moldability and film-forming properties; fillers, crosslinking agents or curing agents aimed at improving the mechanical properties of the molded product; dispersants aimed at imparting dispersibility; pigments and dyes aimed at coloring; thermal stabilizers or light stabilizers aimed at imparting durability; plasticizers aimed at improving flexibility; and (reactive) solvents aimed at improving fluidity before shaping, among others. Further, the composition of the present invention may additionally comprise other antifouling substances against marine fouling organism.

The composition of the invention can be suitably used as, for example, paints and materials for ships, paints and materials for marine-related facilities such as power generation and transmission facilities, port facilities, navigational aid facilities, observation facilities, and aquariums, as well as fishing gear including aquaculture.

In another aspect, the present invention relates to a method for preventing the adhesion of marine fouling organisms, which comprises applying the dipeptide or the tripeptide of the present invention.

In further other aspect, the present invention relates to the dipeptide or the tripeptide of the present invention for preventing the adhesion of marine fouling organisms.

### Examples

Hereinafter, the present invention will be described in more detail by way of the working examples, but it should be noted that these would not limit the scope of the present invention and merely illustrate the invention.

### Overview of the synthesis of dipeptides or tripeptides

Using dipeptide 1 composed of proline and phenylalanine, which is commercially available, as a parent skeleton, various esters of α-amino acids, β-amino acids, or γ-amino acids were condensed to synthesize novel tripeptides 2 to 10. In dipeptide 1, the amino group of phenylalanine is protected by a tert-butyloxycarbonyl group (Boc group).

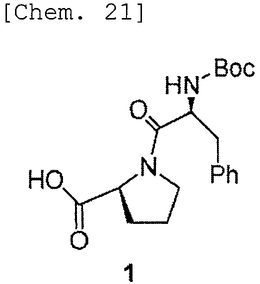

Tripeptide ethyl esters 2 to 5 were synthesized using the very inexpensive amino acid ethyl ester hydrochlorides.

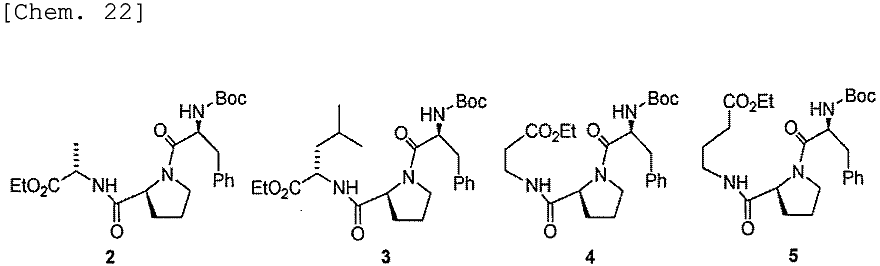

Tripeptide benzyl ester 6 and tripeptide 7 were synthesized using the β-amino acid benzyl ester, which the inventors synthesized (as part of the first step in the synthesis scheme of dipeptide 11) to examine the effects of β-amino acids at this introduction site. 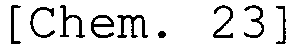 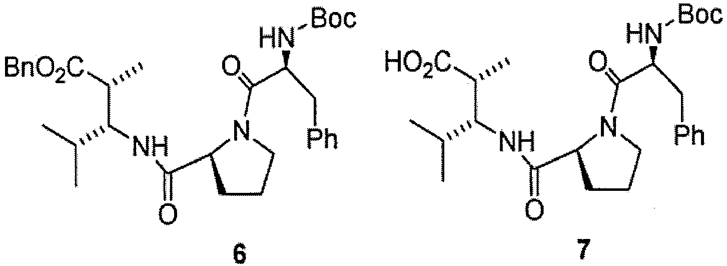 in which Bn is a benzyl group.

Furthermore, using commercially available β-amino acids, benzyl esters and ethyl esters 8 to 10 were synthesized.

Additionally, dipeptide 11 consisting of the β-amino acid ester and proline was synthesized.

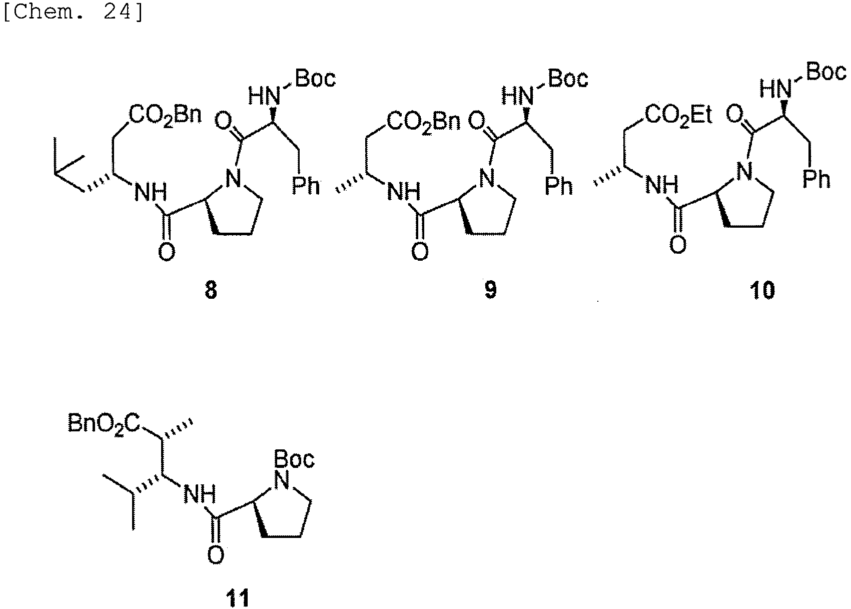

Hereinafter, the synthesis examples of tripeptides 2 to 11 are described in detail.

### (Synthesis of tripeptide 8)

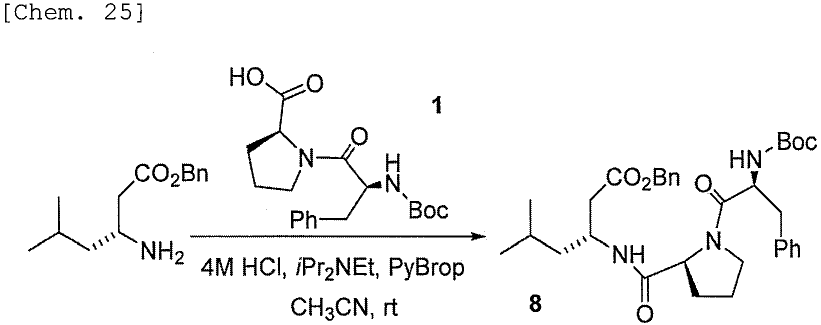

After 16 mg of the β-amino acid benzyl ester as shown above was dissolved in 0.3 mL of acetonitrile, 0.4 mL of a solution of 36 mg of dipeptide 1 in acetonitrile, 0.035 mL of diisopropylethylamine, 0.017 mL of a 4M hydrochloric acid in dioxane solution, and 55 mg of bromotripyrrolidino phosphonium hexafluorophosphate (PyBroP) were added thereto, and the mixture was stirred for 6 hours. A diluted aqueous solution of sodium hydroxide was added to the reaction solution, the mixture was extracted with ethyl acetate, and the extract was purified by silica gel chromatography (hexane: ethyl acetate = 90:10 to 70:30), to yield 37 mg of tripeptide compound 8 targeted. The NMR data and Mass spectrometry data of compound 8 are shown below.
¹H NMR (400 MHz, CDCl₃, as a mixture of 1:1 rotational isomers) δ 0.82-0.91 (m, 6H), 1.16-1.61 (m, 13H), 1.83-2.26 (m, 4H), 2.45-2.62 (m, 2H), 2.83-2.93 (m, ¹H), 3.02 (dd, J = 13.9, 6.1 Hz, 0.5H), 3.14-3.20 (m, 0.5H), 3.39-3.60 (m, 2H), 4.27-4.34 (m, 1.5H), 4.44-4.46 (m, 0.5H), 4.64 (q, J = 7.8 Hz, 0.5H), 5.02-5.17 (m, 2H), 5.28 (d, J = 8.3 Hz, 0.5H), 6.93 (d, J = 8.8 Hz, 0.5H), 7.17-7.38 (m, 10H), 7.49 (d, J = 9.3 Hz, 0.5H)
¹³C NMR (101 MHz, CDCl₃) δ 21.6, 21.8, 22.1, 22.8, 23.1, 24.9, 25.0, 27.4, 28.2, 28.3, 30.5, 38.8, 39.0, 39.2, 39.4, 42.7, 43.1, 44.5, 45.1, 46.4, 47.3, 53.2, 54.1, 60.2, 60.9, 66.2, 66.3, 79.7, 80.2, 126.8, 127.4, 128.1, 128.2, 128.2, 128.3, 128.4, 128.5, 128.8, 129.3, 129.3, 135.7, 135.9, 136.3, 155.1, 155.6, 169.8, 170.0, 171.2, 171.3, 171.4, 171.9.
HRMS (ESI) m/z: [M + Na]⁺ Anal. Calcd for C₃₃H₄₅N₃O₆Na: 602.3201; Found: 602.3228.

### [Synthesis of tripeptides 2 to 5]

Tripeptides 2 to 5 were synthesized in the same manner as that in tripeptide 8, except for a change of the β-amino acid benzyl ester.

### [Synthesis of tripeptides 6, 9, and 10]

Tripeptides 6, 9, and 10 were synthesized in the same manner as that in tripeptide 8, except for a change of the β-amino acid benzyl ester.

### Synthesis of tripeptide 7

Tripeptide 7 was synthesized in the same manner as that in tripeptide 8, except that the β-amino acid benzyl ester was changed to the β-amino acid.

### [Synthesis of dipeptide 11]

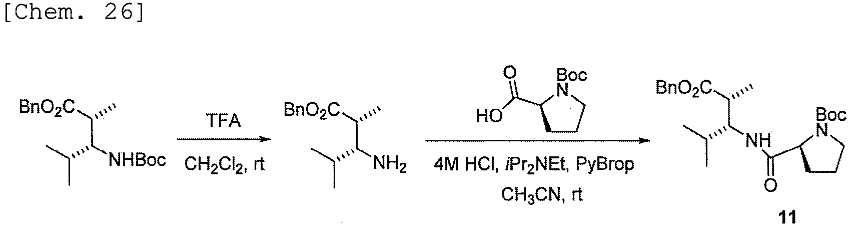

After 36 mg of the known Boc-β-amino acid benzyl ester was dissolved in 0.6 mL of dichloromethane, 0.2 mL of trifluoroacetic acid was added thereto, and the mixture was stirred for 40 minutes. A diluted aqueous solution of sodium hydroxide was added to the reaction solution, and the mixture was extracted with dichloromethane to yield the β-amino acid benzyl ester.

After the β-amino acid benzyl ester as shown above was dissolved in 0.6 mL of acetonitrile, 25 mg of Boc-proline, 0.055 mL of diisopropylethylamine, 0.026 mL of a 4M hydrochloric acid in dioxane solution, and 25 mg of bromotripyrrolidino phosphonium hexafluorophosphate (PyBroP) were added thereto, and the mixture was stirred for 6 hours. A diluted aqueous solution of sodium hydroxide was added to the reaction solution, the mixture was extracted with ethyl acetate, and the extract was purified by silica gel chromatography (hexane:ethyl acetate = 90:10 to 70:30), to yield 32 mg of dipeptide 11 targeted. The NMR and Mass spectrometry data of compound 11 are shown below.
¹H NMR (400 MHz, CDCl₃) δ 0.89 (3H, d, J = 6.6 Hz), 0.92 (3H, d, J = 6.5 Hz), 1.16 (3H, d, J = 7.1 Hz), 1.46 (9H, s), 1.62-1.86 (3H, m), 1.90-2.20 (2H, m), 2.85 (¹H, brs), 3.46 (2H, br), 3.77 (¹H, brs), 4.31 (¹H, brs), 5.07 (¹H, d, J = 12.3 Hz), 5.13 (¹H, d, J = 12.3 Hz), 6.83-7.05 (¹H, m), 7.26-7.36 (5H, m);
¹³C NMR (101 MHz, CDCl₃) δ 15.8, 15.9, 19.5, 19.8, 28.3, 31.2, 31.7, 39.9, 40.4, 46.9, 56.7, 61.6, 66.3, 128.2, 128.3, 128.6, 135.6, 154.9, 175.3.
HRMS (ESI) m/z: [M + Na]⁺ Anal. Calcd for C₂₄H₃₆N₂O₅Na: 455.2516; Found: 455.2512.

### [Synthesis of tripeptide 12]

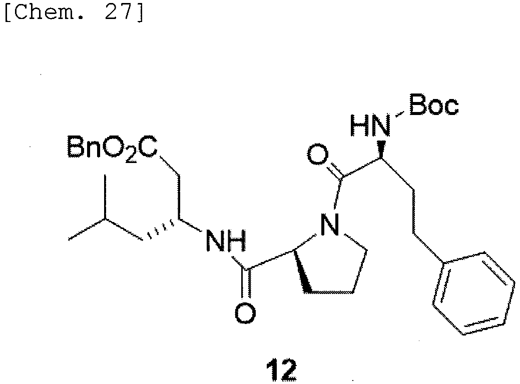

Tripeptide 12 was synthesized in the same manner as that in tripeptide 8, except that the β-amino acid benzyl ester was changed to the β-amino acid.

The NMR and Mass spectrometry data of compound 12 are shown below.
¹H NMR (500 MHz, CDCl₃) δ 0.86 (6H, d, J = 6.6 Hz), 1.21-1.27 (¹H, m), 1.40-1.47 (¹H, m), 1.45 (9H, s), 1.55-1.59 (¹H, m), 1.84-2.04 (5H, m), 2.25 (¹H, t, J = 8.5 Hz), 2.44-2.53 (2H, m), 2.66-2.76 (2H, m), 3.27-3.31 (¹H, m), 3.46-3.49 (¹H, m), 4.25-4.30 (¹H, m), 4.42-4.52 (2H, m), 4.98 (¹H, d, J = 12.3 Hz), 5.05 (¹H, d, J = 12.3 Hz), 5.31 (¹H, d, J = 8.7 Hz), 6.94 (¹H, d, J = 9.0 Hz), 7.15-7.36 (10H, m);
¹³C NMR (126 MHz, CDCl₃) δ 21.9, 22.9, 24.9, 27.5, 28.3, 31.5, 34.7, 39.1, 43.1, 44.2, 46.9, 51.2, 60.0, 66.2, 79.6, 126.0, 128.2, 128.39, 128.46, 128.47, 128.60, 135.6, 140.9, 155.5, 170.2, 171.3, 172.4;
HRMS (ESI) m/z: [M + Na]⁺ Anal. Calcd for C₃₄H₄₇N₃O₆Na: 616.3357; Found: 616.3342.

### [Synthesis of dipeptide 14]

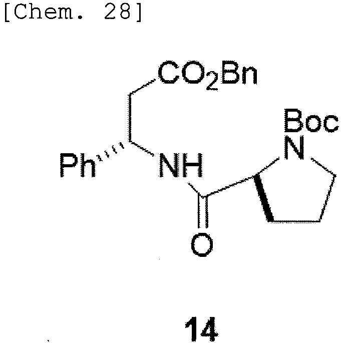

Dipeptide 14 was synthesized in the same manner as that in dipeptide 11, except for a change of the Boc-β-amino acid benzyl ester.

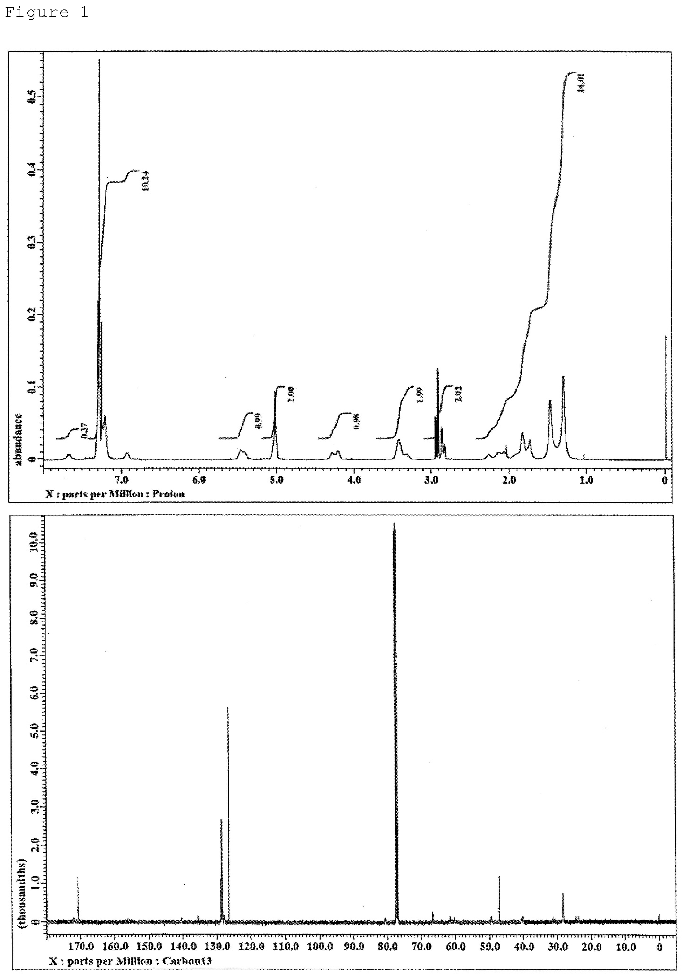

The spectra ¹H NMR (500 MHz, CDCl₃) and ¹³C NMR (126 MHz, CDCl₃) of compound 14 are shown in Figure 1. both of the ¹H NMR (top) and ¹³C NMR (bottom) of Figure 1 indicate that compound 14 is a mixture of rotational isomers that cannot be analyzed. The Mass spectrometry data of compound 14 is as follows.
HRMS (ESI) m/z: [M + Na]⁺ Anal. Calcd for C₂₆H₃₂N₂O₅Na: 475.2203; Found: 475.2205.

### Reference Example 1

### Synthesis of dipeptide 13

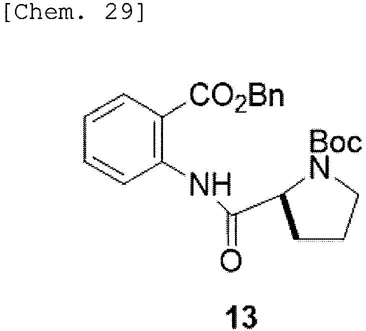

Dipeptide 13 was synthesized in the same manner as that in dipeptide 11, except for a change of the Boc-β-amino acid benzyl ester. Dipeptide 13 is a compound described in Tetrahedron, 2011, 67, 3969, wherein the compound is merely mentioned as a model substance for asymmetric reactions.
¹H NMR (500 MHz, CDCl₃, as a mixture of 1.5:1 rotational isomers) δ 1.38 (9H×1.5/2.5, s), 1.50 (9H×1/2.5, s), 1.92-2.35 (4H, m), 3.45-3.80 (2H, m), 4.28-4.30 (¹H×1.5/2.5, m), 4.58-4.66 (¹H×1/2.5, m) 5.33 (2H, s), 7.02-7.11 (¹H, m), 7.32-7.44 (5H, m), 7.49-7.61 (¹H, m), 8.05-8.12 (¹H, m), 8.72-8.80 (¹H, m), 11.54 (¹H×1.5/2.5, s), 11.60 (1H×1/2.5, s) ;
¹³C NMR (126 MHz, CDCl₃) δ 23.8, 24.3, 28.0, 28.2, 28.4, 30.5, 31.5, 46.8, 47.1, 62.1, 62.7, 66.7, 67.0, 80.1, 80.3, 115.2, 120.0, 120.3, 122.5, 122.6, 128.1, 128.3, 128.4, 128.6, 130.7, 130.9, 134.6, 134.7, 135.4, 141.1, 154.2, 167.6, 172.4;
HRMS (ESI) m/z: [M + Na]⁺ Anal. Calcd for C₂₄H₂₈N₂O₅Na: 447.1890; Found: 447.1901.

### Text Example 1

### Evaluation of antifouling activities and toxicity against Striped Barnacle (1)

### Evaluation of antifouling activities and toxicity against the cypris larvae of Striped Barnacle

The antifouling activities of the synthesized tripeptides against the cypris larvae of Striped Barnacle were evaluated.

After solutions of about 2 mL of the test compounds (tripeptides 2 to 10 and dipeptide 11) in ethanol were dispensed into each well of the 24-well polystyrene multi-well plate (3.2 mL/well, well diameter 15.5 mm, well height 17.6 mm, manufactured by Corning), the multi-well plate was air-dried to evaporate the ethanol.

The test compounds were aliquoted to achieve final concentrations of 0.03, 0.1, 0.3, 1, 3, and 10 µg/mL.

Subsequently, six individuals of the cypris larvae of Striped Barnacle (hereinafter simply referred to as "barnacle larvae") were transplanted into each well along with 2 mL of pre-filtered natural seawater. The multi-well plate was left standing in an incubator at 25°C, and the barnacle larvae were allowed to grow for two days at 25°C.

In the above procedure, a test for the control group was conducted by dispensing an ethanol solution without any test compound. The natural seawater used in the above test was prepared by diluting the natural seawater collected from the coastal area near Onjuku, Chiba Prefecture (salt concentration: 36 g/kg) with distilled water to adjust a salt concentration of 32 g/kg.

Additionally, in the above procedure, a test for the comparison group was conducted by dispensing the copper sulfate solution for comparison.

In the test group, the control group, and the comparison group, the tests were conducted in four times (four wells) for one concentration of each test compounds.

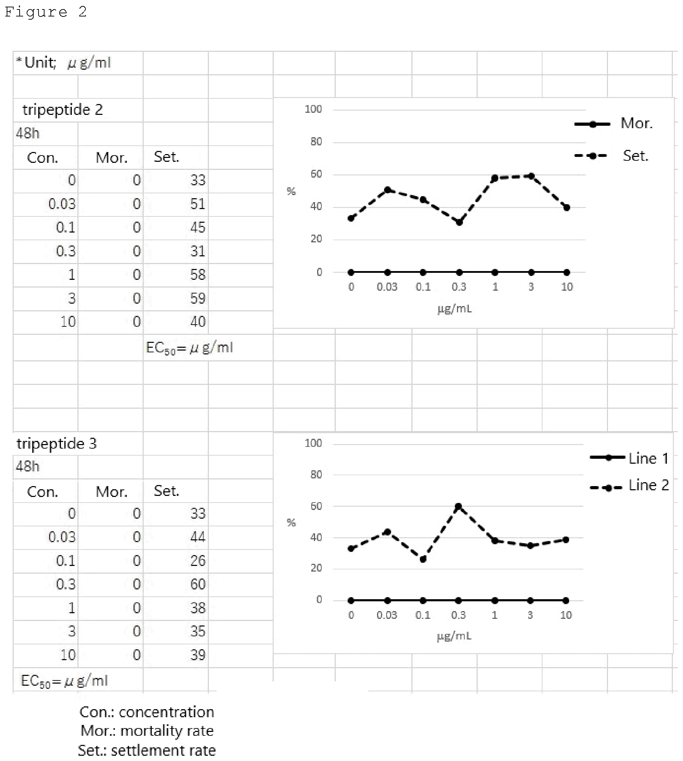

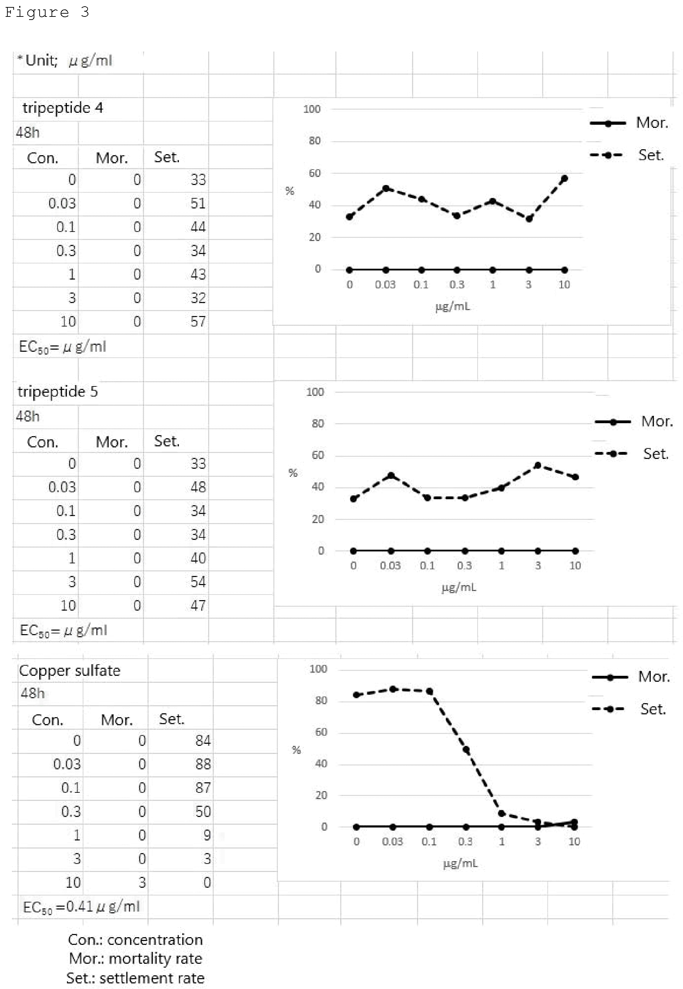

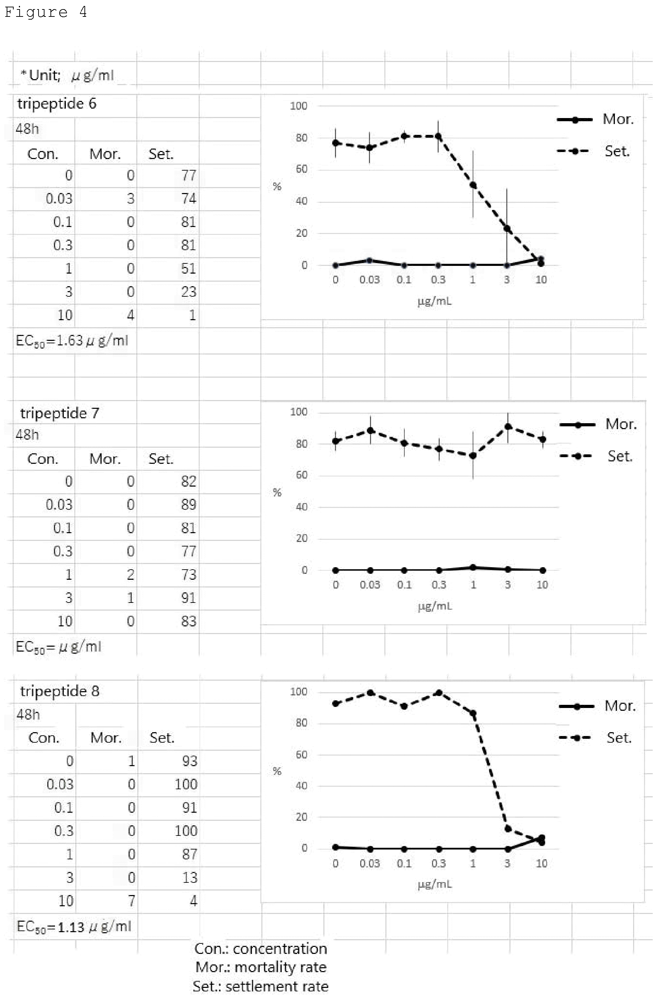

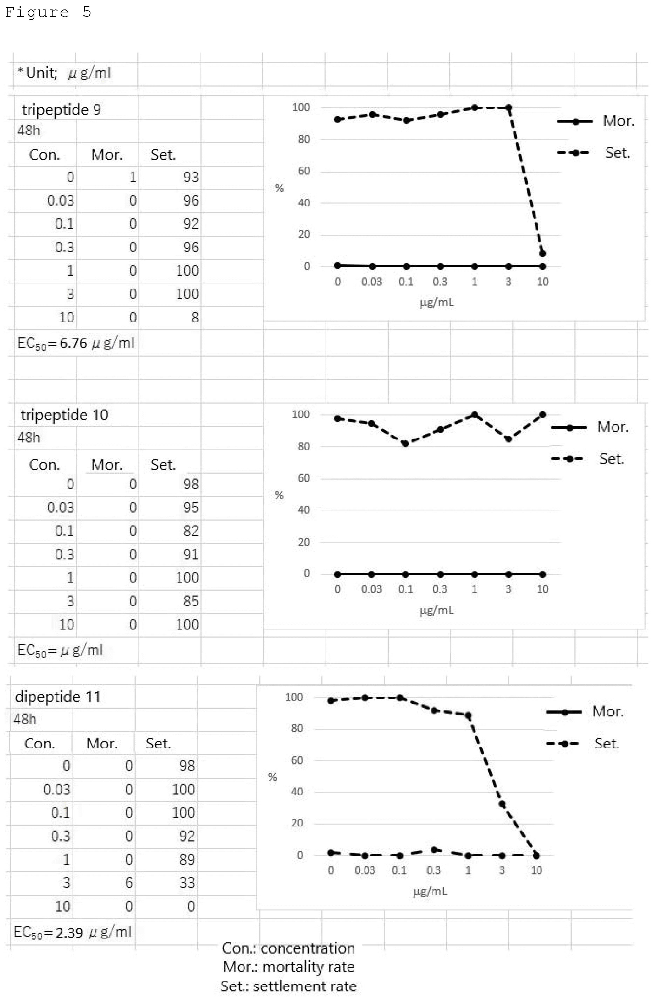

Forty-eight hours after the test started, the number of adhered individuals and the number of dead individuals of the barnacle larvae in each well were counted under a stereomicroscope. The adhesion rate (%) and mortality rate (%) of the barnacle larvae in each well were calculated, respectively. For each test compound, a dose-adhesion response curve was created, and the half-maximal effective concentration (48h EC₅₀) that prevents the adhesion of the barnacle larvae by 50% was determined. Similarly, for each test compound, a dose-mortality response curve was created to determine the half-maximal lethal concentration (48h LC₅₀) that causes 50% mortality in the barnacle larvae. The results are shown in Table 1 and Figures 1 to 5. As a reference, the value of Boc-3 obtained from non-Patent literature 8 is also described.

In Figures 1 to 5, "mortality rate" refers to a ratio of the number of dead individuals to the total number of the individuals, while "settlement rate" refers to a ratio of the number of individuals that transformed from larvae to adhered adults to the total number of the individuals. In particular, the lower the settlement rate, the stronger the antifouling effect.

**[Table 1]**

| | **Test compounds** | **Antifouling activity 48h EC₅₀ (*µ*g/mL)** | **Toxicity 48h LC₅₀ (*µ*g/mL)** |
|---|---|---|---|
| **Control group** | **---** | **---** | **---** |
| **Test group** | **tripeptide 2** | **>10** | **>10** |
| | **tripeptide 3** | **>10** | **>10** |
| | **tripeptide 4** | **>10** | **>10** |
| | **tripeptide 5** | **>10** | **>10** |
| | **tripeptide 6** | **1.63** | **>10** |
| | **tripeptide 7** | **>10** | **>10** |
| | **tripeptide 8** | **1.13** | **>10** |
| | **tripeptide 9** | **6.76** | **>10** |
| | **tripeptide 10** | **>10** | **>10** |
| | **dipeptide 11** | **2.39** | **>10** |
| **Comparison group** | **Copper sulfate** | **0.41** | **>10** |
| **Reference ¹⁾** | **Boc-3** | **0.79** | **>10** |

| | | | |
|---|---|---|---|
| **1) See Table 1 in non-Patent literature 8** | | | |

Tripeptides 2 to 5, which were synthesized from inexpensive amino acids such as alanine, leucine, β-alanine, and GABA, did not exhibit any antifouling activity within the tested concentration range.

Tripeptide 6, in which the β-amino acid ester (synthetic product) contained in the reference tripeptide was introduced, exhibited moderate antifouling activity, but, tripeptide 7, in which the C-terminus end is a carboxylic acid instead of an ester, exhibited no antifouling activity. From this, it was considered that the presence of the β-amino acid ester at this position is important for adhesion prevention.

Based on this consideration, the benzyl ester (Bn) or ethyl ester (Et) derived from the commercially available β-amino acids was introduced into the tripeptides. As a result, the benzyl ester exhibited an antifouling activity, while the ethyl ester did not. The difference between these esters is understood to be the difference in the hydrophobicity of the substituents.

Moreover, there were hardly any individuals that died in these tests.

### Text Example 2

### Evaluation of the antifouling activity and toxicity against the juvenile shellfish of Mediterranean mussel

Tripeptides 8 to 10 were evaluated for their antifouling activity against the juvenile shellfish of Mediterranean mussel.

The sample was dissolved in ethanol to adjust to a concentration of 1 mg/mL. Thirty and 100 µL (30 and 100 µg) of the sample solutions were mixed with the solvent, and the mixtures were brought to a total volume of 500 µL. These mixtures were spread on the bottom surface of the glass beaker of the test container, and the solvent was volatilized by shaking them overnight. As a result, the samples to be evaluated were coated only on the bottom surface of the glass beaker.

A volume of 10 mL of the test seawater was added to the glass beakers of the test containers. The tests were initiated by introducing twelve test individuals into the test seawater in each beaker. The tests ended after three days, and the observation was conducted.

At the end of the tests conducted at two concentrations of 3.0 µg/mL and 10 µg/mL, the results of the condition of juvenile Mediterranean mussels and the number of byssal threads were shown in Table 2. Table 2 shows the proportion of individuals that did not adhere at 3.0 µg/mL (the overturned individuals and the closed shell individuals in Table 2).

In Table 2, the terms "bottom surface-adhered individuals" and "wall surface-adhered individuals" refer to individuals that are adhered to the bottom surface and wall surface of the beaker, respectively; the term "bottom surface-crawling individuals" refers to individuals that move along the bottom surface without adhering; the term "overturned individuals or closed shell individuals (unadhered)" refers to individuals that are either overturned or in a closed shell without adhering to the bottom surface; and the term "number of byssal threads on the bottom surface" refers to the number of foot threads that are fixed to the bottom surface. In particular, the greater the reduction in the number of byssal threads, the stronger the effect of adhesion prevention.

Moreover, there were no deceased individuals in these tests.

**[Table 2]**

| **Compounds** | **Reactions and conditions of the larvæ during observation** | **3 µg/mL (30 *µ*g)** | | **10 µg/mL (100 *µ*g)** | |
|---|---|---|---|---|---|
| | | **No.** | **%** | **No.** | **%** |
| **Control group** | **bottom surface-adhered individuals** | **5** | **41.7** | **-**-- | **---** |
| | **bottom surface-crawling individuals** | **0** | **0.0** | **-**-- | **---** |
| | **wall surface-adhered individuals** | **7** | **58.3** | **-**-- | **---** |
| | **overturned individuals or closed shell individuals (unadhered)** | **0** | **0.0** | **-**-- | **---** |
| | **number of byssal threads on the bottom surface** | | **118** | **-**-- | **---** |
| **8** | **bottom surface-adhered individuals** | **2** | **16.7** | **2** | **16.7** |
| | **bottom surface-crawling individuals** | **0** | **0.0** | **0** | **0.0** |
| | **wall surface-adhered individuals** | **3** | **25.0** | **0** | **0.0** |
| | **overturned individuals or closed shell individuals (unadhered)** | **7** | **58.3** | **10** | **83.3** |
| | **number of byssal threads on the bottom surface** | | **25** | | **12** |
| **9** | **bottom surface-adhered individuals** | **9** | **75.0** | **7** | **58.3** |
| | **bottom surface-crawling individuals** | **0** | **0.0** | **0** | **0.0** |
| | **wall surface-adhered individuals** | **1** | **8.3** | **1** | **8.3** |
| | **overturned individuals or closed shell individuals (unadhered)** | **2** | **16.7** | **4** | **33.3** |
| | **number of byssal threads an the bottom surface** | | **73** | | **34** |
| **10** | **bottom surface-adhered individuals** | **8** | **66.7** | **7** | **58.3** |
| | **bottom surface-crawling individuals** | **0** | **0.0** | **0** | **0.0** |
| | **wall surface-adhered individuals** | **3** | **25.0** | **4** | **33.3** |
| | **overturned individuds or closed shell individuals (unadhered)** | **1** | **8.3** | **1** | **8.3** |
| | **number of byssal threads on the bottom surface** | | **80** | | **11.5** |
| **Dolastin 16** | **bottom surface-adhered individuals** | **9** | **75.0** | **12** | **100.0** |
| | **bottom surface-crawling individuals** | **0** | **0.0** | **0** | **0.0** |
| | **wall surface-adhered individuals** | **3** | **25.0** | **0** | **0.0** |
| | **overturned individuals or closed shell individuals (unadhered)** | **0** | **0.0** | **0** | **0.0** |
| | **number of byssal threads on the bottom surface** | | **72** | | **45** |
| **Boc-3** | **bottom surface-adhered individuals** | **3** | **25.0** | **1** | **8.3** |
| | **bottom surface-crawling individuals** | **3** | **25.0** | **0** | **0.0** |
| | **wall surface-adhered individuals** | **2** | **16.7** | **4** | **33.3** |
| | **overturned individuals or closed shell indviduals (un adhered)** | **4** | **33.3** | **7** | **58.3** |
| | **number of byssal threads on the bottom surface** | | **23** | | **18** |
| **Copper sulfate** | **bottom surface-adhered individuals** | **0** | **0.0** | **-**-- | **---** |
| | **bottom surface-crawling individuals** | **0** | **0.0** | **-**-- | **-**-- |
| | **wall surface-adhered individuals** | **0** | **0.0** | **-**-- | **-**-- |
| | **overturned individuals or dosed shell individuals (unadhered)** | **12** | **100.0** | **-**-- | **-**-- |
| | **number of byssal threads on the bottom surface** | | **2** | **-**-- | **-**-- |

### [Control group]

The number of byssal threads was large, and all individuals were adhered to the bottom surface or the wall surface.

Therefore, it was valid as a control group, and the test was successful.

### [tripeptide 8]

The adhesion to the bottom surface was observed, but the number of the individuals was small.

The numbers of the overturned individuals and the closed shell individuals were large, and were increased due to the rise in concentration.

Due to the rise in concentration, the number of byssal threads was decreased.

Therefore, tripeptide 8 is determined to exhibit an antifouling effect.

### [tripeptide 9]

The adhesion to the bottom surface was observed.

Due to the rise in concentration, the number of the bottom surface-adhered individuals was decreased, the number of overturned individuals was increased, and the number of byssal threads was decreased.

Therefore, compound 9 is concentration-dependent and may be determined to be effective.

### [tripeptide 10]

The adhesion to the bottom surface was observed.

Due to the rise in concentration, the number of the bottom surface-adhered individuals and the number of overturned individuals remained unchanged, and the number of the byssal threads was increased.

Therefore, compound 10 may be determined to have no concentration dependence and to be ineffective.

### [Dolastatin 16]

Adhesion to the bottom surface was observed, and the number of individuals was large.

Due to the rise in concentration, the number of the bottom surface-adhered individuals was not decreased, the number of the overturned individuals was not increased, and the number of the byssal threads were decreased.

Therefore, Dolastatin 16 is determined to exhibit no antifouling effect.

### [Boc-3]

Adhesion to the bottom surface was observed, but the number of individuals was small.

Due to the rise in concentration, the number of the bottom surface-adhered individuals was decreased, the number of the overturned individuals was increased, and the number of the byssal threads was decreased.

Therefore, Boc-3 may be determined to be concentration-dependent, and to be effective.

### [Copper sulfate]

No adhered individuals were observed on the bottom surface nor the wall surface, and all were either the overturned individuals or the closed shell individuals.

Only a few instances of the byssal threads were observed.

The antifouling effect was high.

### [General]

It was very interesting that the above results corresponded to the evaluation on the cypris larvae of Striped Barnacle. That is, compound 8 was effective against both the barnacle and Mediterranean mussel.

### Text Example 3

### Evaluation of the antifouling activity and toxicity against Mediterranean mussel (2)

Tripeptide 12, dipeptide 13, and dipeptide 14 were tested in the same manner as in Test Example 1, so as to evaluate their antifouling activity and toxicity.

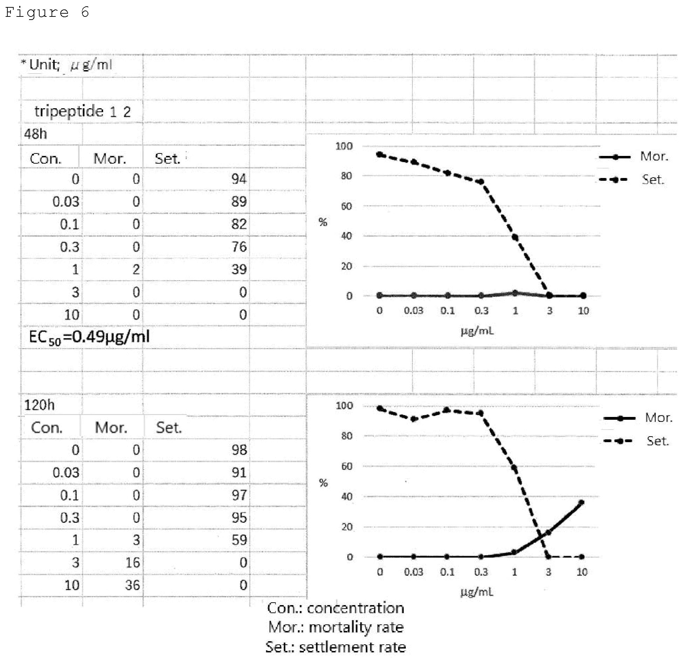

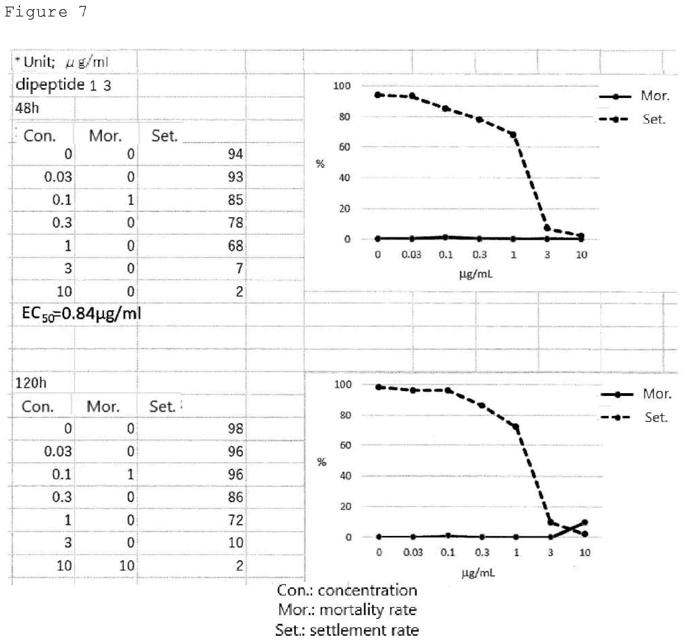

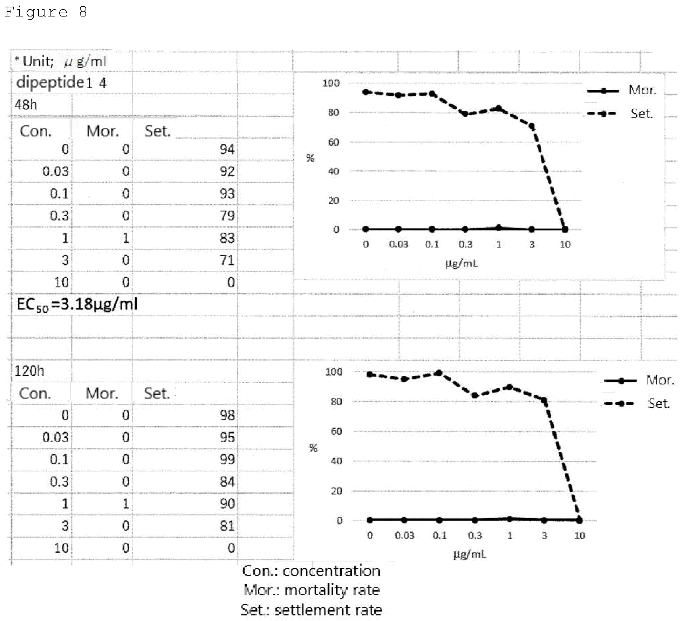

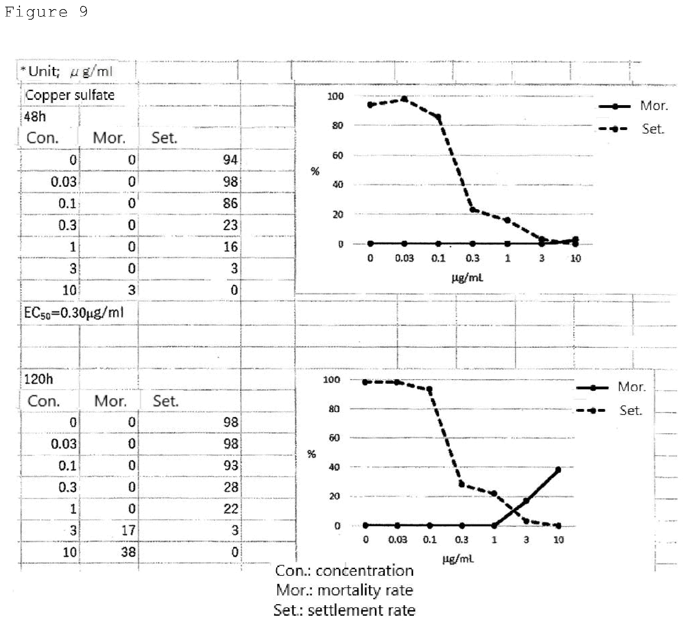

The obtained results are shown in Table 3 and Figures 6 to 9, along with the results of the copper sulfate, which has been recognized for its antifouling activity.

**[Table 3]**

| | **Test compounds** | **Antifouling activity** | **Toxicity** |
|---|---|---|---|
| | | **48h EC₅₀ (*µ*g/mL)** | **48h LC₅₀ (*µ*g/mL)** |
| **Control group** | **---** | **---** | **---** |
| **Test group** | **tripeptide 12** | **0.49** | **>10** |
| | **dipeptide 13** | **0.84** | **>10** |
| | **dipeptide 14** | **3.18** | **>10** |
| **Comparison group** | **Copper sulfate** | **0.3** | **>10** |

In one aspect, the present invention relates to a composition for preventing the adhesion of marine fouling organisms, which comprises a dipeptide containing a proline residue, or a tripeptide containing a proline residue and a phenylalanine residue, that is represented by formula (1): 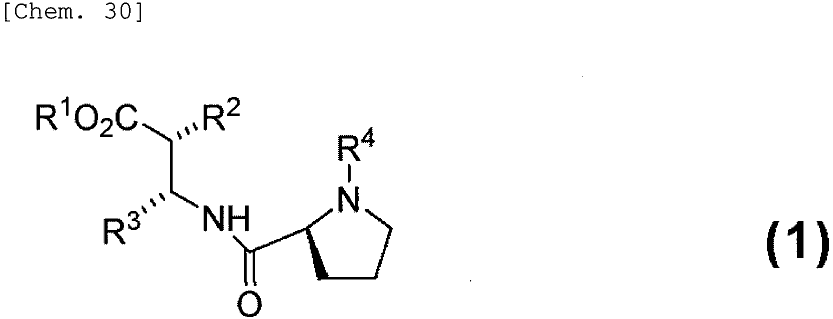 in which
R1, R2, R3, and R4 are the same meaning as above.

The relationships between the cumulative carbon number in substituents: R1, R2, and R3 of the compound of the present invention and the antifouling effect against marine fouling organisms are summarized in Table 4 based on the results from Test Examples 1 to 3.

**[Table 4]**

| | **Compounds** | **R1** | **R2** | **R3** | **Cumulative carbon number** | **Antifouling activity 48h EC₅₀ (*µ*g/mL)** | **Claim scope** |
|---|---|---|---|---|---|---|---|
| **Tri** | **2** | **Et** | **Me** | **none** | **3** | **>10** | **Outside** |
| | **3** | **Et** | **i-Bu** | **none** | **6** | **>10** | **Outside** |
| | **4** | **Et** | **H** | **H** | **2** | **>10** | **Outside** |
| | **5** | **Et** | **H** | **H, CH2-** | **3** | **>10** | **Outside** |
| | **6** | **Bn** | **Me** | **i-Pr** | **12** | **1.66** | **Inside** |
| | **7** | **H** | **Me** | **i-Pr** | **4** | **>10** | **Outside** |
| | **8** | **Bn** | **H** | **i-Bu** | **11** | **1.1** | **Inside** |
| | **9** | **Bn** | **H** | **Me** | **8** | **6.8** | **Inside** |
| | **10** | **Et** | **H** | **Me** | **3** | **>10** | **Outside** |
| | **12** | **Bn** | **H** | **i-Bu** | **11** | **0.5** | **Inside** |
| **Di** | **11** | **Bn** | **Me** | **i-Pr** | **11** | **2.4** | **Inside** |
| | **13** | **Bn** | | **Ph** | **11** | **0.8** | **Inside** |
| | **14** | **Bn** | **H** | **Ph** | **13** | **3.2** | **Inside** |
| **Claim1** | | **3~10** | **0~8** | **0~8** | **3-26** | | |

Table 4 shows that, from the perspective of the cumulative carbon numbers of R1, R2, and R3, up to 6 provided weak effects, around 8 provided definite activities, and beyond 10 provided enhanced activities.

The treatment showed no toxicity and achieved an antifouling effect against both the barnacle larvae and the mussels, which adhere in different manners to marine structures and ship hulls.

Accordingly, promising candidates for effective antifouling substances against various fouling organisms present in the ocean can be provided.

### INDUSTRIAL APPLICABILITY

The present invention enables the synthesis of compositions (dipeptides composed of a β-amino acid ester and a proline derivative, and tripeptides composed of a β-amino acid ester, a proline derivative, and a phenylalanine derivative) that prevent the adhesion of marine fouling organisms in a cost-effective and simple manner, with low environmental impact.

## Claims

1. A dipeptide containing a proline residue, or a tripeptide containing a proline residue and a phenylalanine residue represented by formula (1): 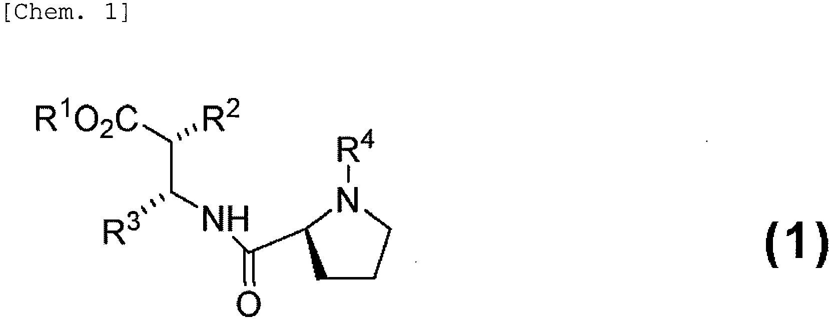 in which
R1 is a substituted or unsubstituted C3 to C10 hydrocarbon group;
R2 and R3 are a hydrogen atom or a substituted or unsubstituted C1 to C8 hydrocarbon group, or R2 and R3 together with the adjacent carbon atoms form a substituted or unsubstituted cyclic C1 to 10 hydrocarbon group;
R4 is a hydrogen atom, a substituted or unsubstituted C1 to C6 hydrocarbon group, a carboxyl group or alkoxy acyl group represented by formula (2): 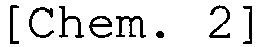 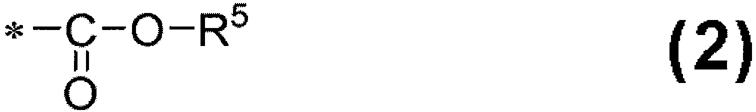 in which
R5 is a hydrogen atom or a C1 to C6 hydrocarbon group, and the symbol * indicates the bonding part with the pyrrolidine group, or a phenylalanine residue represented by formula (3) or (3'); 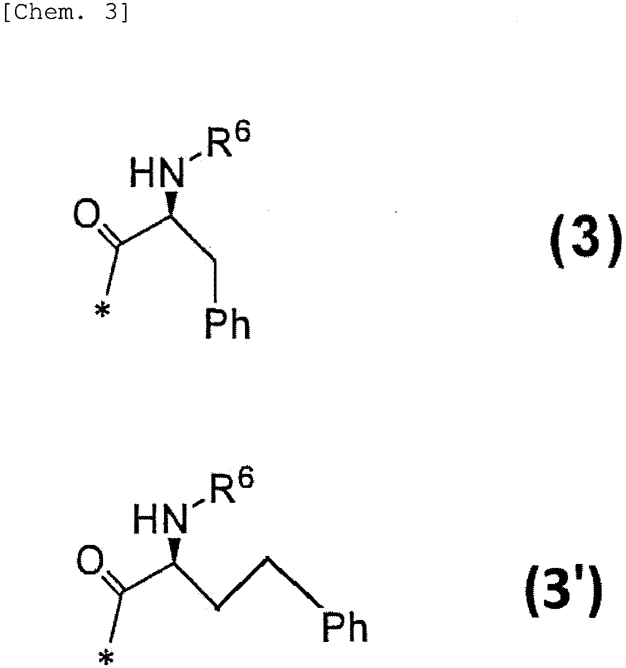 in which
R6 is a hydrogen atom, a C1 to C6 hydrocarbon group, or a carboxyl group or alkoxy acyl group represented by formula (2), Ph is a phenyl group, and the symbol * indicates the bonding part with the pyrrolidine group;
provided that compounds in which R1 is a benzyl group, both R2 and R3 are phenyl groups, and R4 is a tert-butoxycarbonyl group are excluded.

2. The dipeptide containing the proline residue, or the tripeptide containing the proline residue and the phenylalanine residue according to claim 1, wherein the cumulative carbon number in substituents: R1, R2, and R3 is 7 or more, preferably 9 or more, and more preferably 11 or more.

3. The dipeptide containing the proline residue, or the tripeptide containing the proline residue and the phenylalanine residue according to claim 1,
in which
the substituted or unsubstituted C3 to C10 hydrocarbon group in R1 is selected from the group consisting of a substituted or unsubstituted, linear or branched C3 to C10 alkyl group; a substituted or unsubstituted, linear or branched C3 to C10 alkenyl group; a substituted or unsubstituted cyclic C3 to C10 alkyl group; a substituted or unsubstituted cyclic C3 to C10 alkenyl group; and a substituted or unsubstituted C3 to C10 alkyl-substituted aryl group;
the substituted or unsubstituted C1 to C8 hydrocarbon groups in R2 and R3 are independently selected from the group consisting of a substituted or unsubstituted, linear or branched C1 to C8 alkyl group; a substituted or unsubstituted, linear or branched C1 to C8 alkenyl group; a substituted or unsubstituted cyclic C1 to C8 alkyl group; a substituted or unsubstituted cyclic C1 to C8 alkenyl group; and a substituted or unsubstituted C1 to C8 alkyl-substituted aryl group, and, when R2 and R3 together with the adjacent carbon atoms form a substituted or unsubstituted cyclic C1 to C10 hydrocarbon group, the cyclic C1 to C10 hydrocarbon group is selected from the group consisting of a substituted or unsubstituted cyclic C1 to C10 alkyl group; a substituted or unsubstituted cyclic C1 to C10 alkenyl group; and a substituted or unsubstituted C1 to C10 alkyl-substituted aryl group;
the substituted or unsubstituted C1 to C6 hydrocarbon group in R4 is selected from the group consisting of a substituted or unsubstituted, linear or branched C1 to C6 alkyl group; a substituted or unsubstituted, linear or branched C1 to C6 alkenyl group; a substituted or unsubstituted cyclic C1 to C6 alkyl group; a substituted or unsubstituted cyclic C1 to C6 alkenyl group; and a substituted or unsubstituted C1 to C6 alkyl-substituted aryl group; and
the substituted or unsubstituted C1 to C6 hydrocarbon groups at R5 and R6 are independently selected from the group consisting of a substituted or unsubstituted, linear or branched C1 to C6 alkyl group; a substituted or unsubstituted, linear or branched C1 to C6 alkenyl group; a substituted or unsubstituted cyclic C1 to C6 alkyl group; a substituted or unsubstituted cyclic C1 to C6 alkenyl group; and a substituted or unsubstituted phenyl group.

4. The dipeptide containing the proline residue, or the tripeptide containing the proline residue and the phenylalanine residue according to claim 1,
in which
the substituted or unsubstituted C3 to C10 hydrocarbon group in R1 is selected from the group consisting of a linear or branched alkyl group selected from the group consisting of propyl group, butyl group, pentyl group, hexyl group, heptyl group, octyl group, nonyl group, and decyl group; a linear or branched alkenyl group selected from the group consisting of propenyl group, butenyl group, pentenyl group, hexenyl group, heptenyl group, octenyl group, nonenyl group, and decenyl group; a cyclic alkyl group selected from the group consisting of cyclopropyl group, cyclobutyl group, cyclopentyl group, cyclohexyl group, cycloheptyl group, cyclooctyl group, cyclononyl group, and cyclodecyl group; a cyclic alkenyl group selected from the group consisting of cyclopropenyl group, cyclobutenyl group, cyclopentenyl group, cyclohexenyl group, cycloheptenyl group, cyclooctenyl group, cyclononenyl group, and cyclodecenyl group; an aryl group selected from the group consisting of phenyl group and naphthyl group, an alkyl-substituted phenyl group selected from tolyl group, ethylphenyl group, xylyl group, and benzyl group;
the C1 to C8 hydrocarbon group of the substituted or unsubstituted C1 to C8 hydrocarbon group in R2 and R3 is each independently selected from the group consisting of a linear or branched alkyl group selected from the group consisting of methyl group, ethyl group, propyl group, butyl group, pentyl group, hexyl group, heptyl group, and octyl group; a linear or branched alkenyl group selected from the group consisting of ethenyl group, propenyl group, butenyl group, pentenyl group, hexenyl group, heptenyl group, and octenyl group; a cyclic alkyl group selected from the group consisting of cyclopropyl group, cyclobutyl group, cyclopentyl group, cyclohexyl group, cycloheptyl group, and cyclooctyl group; a cyclic alkenyl group selected from the group consisting of cyclopropenyl group, cyclobutenyl group, cyclopentenyl group, cyclohexenyl group, cycloheptenyl group, and cyclooctenyl group; and an aryl group selected from the group consisting of phenyl group, an alkyl-substituted phenyl group selected from tolyl group, ethylphenyl group, xylyl group, and benzyl group; and
the C1 to C6 hydrocarbon group of the substituted or unsubstituted C1 to C6 hydrocarbon group in R5 and R6 is each independently selected from the group consisting of a linear or branched alkyl group selected from the group consisting of methyl group, ethyl group, propyl group, butyl group, pentyl group, and hexyl group; a linear or branched alkenyl group selected from the group consisting of ethenyl group, propenyl group, butenyl group, pentenyl group, and hexenyl group; a cyclic alkyl group selected from the group consisting of cyclopropyl group, cyclobutyl group, cyclopentyl group, and cyclohexyl group; a cyclic alkenyl group selected from the group consisting of cyclopropenyl group, cyclobutenyl group, cyclopentenyl group, and cyclohexenyl group; and phenyl group.

5. A composition for preventing the adhesion of marine fouling organisms, which comprises a dipeptide containing a proline residue, or a tripeptide containing a proline residue and a phenylalanine residue represented by formula (1): 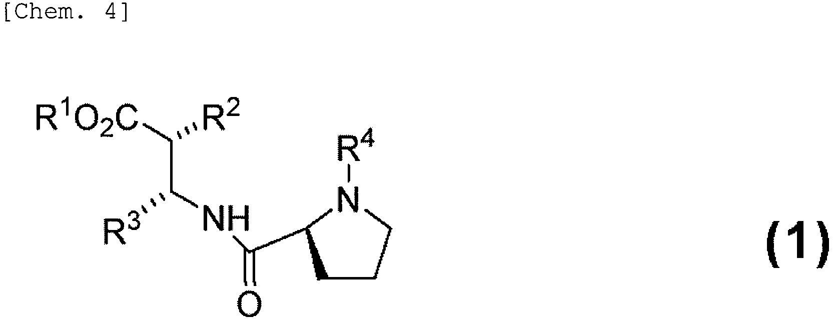 in which
R1 is a substituted or unsubstituted C3 to C10 hydrocarbon group;
R2 and R3 are a hydrogen atom or a substituted or unsubstituted C1 to C8 hydrocarbon group, or R2 and R3 together with the adjacent carbon atoms form a substituted or unsubstituted cyclic C1 to 10 hydrocarbon group;
R4 is a hydrogen atom, a substituted or unsubstituted C1 to C6 hydrocarbon group, a carboxyl group or alkoxy acyl group represented by formula (2): 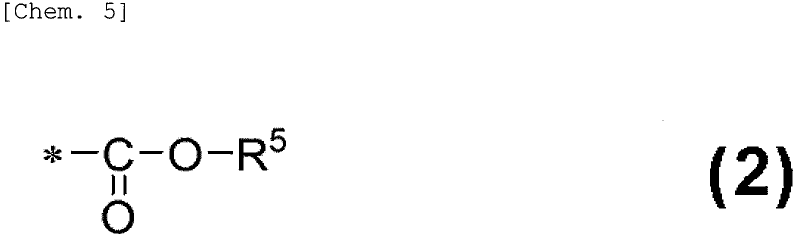 in which
R5 is a hydrogen atom or a C1 to C6 hydrocarbon group, and the symbol * indicates the bonding part with the pyrrolidine group, or a phenylalanine residue represented by formula (3) or (3'); 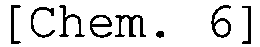 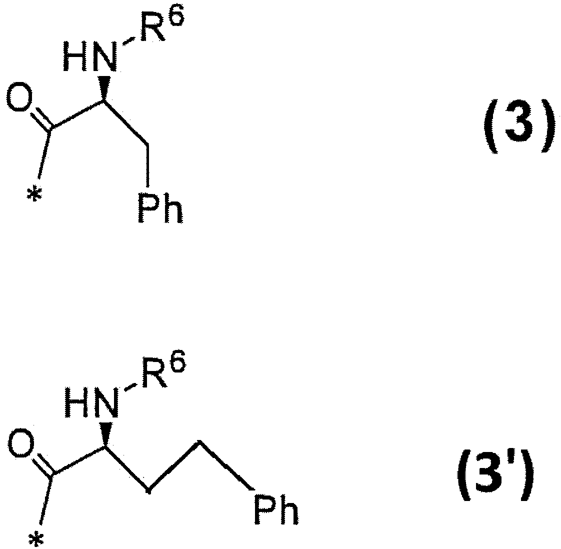 in which
R6 is a hydrogen atom, a C1 to C6 hydrocarbon group, or a carboxyl group or alkoxy acyl group represented by formula (2), Ph is a phenyl group, and the symbol * indicates the bonding part with the pyrrolidine group;
provided that the cumulative carbon number in substituents: R1, R2, and R3 is 7 or more, preferably 9 or more, and more preferably 11 or more.
